# EUROPÄISCHE PATENTANMELDUNG

(11) **EP 3 888 630 A1**
(43) Veröffentlichungstag der Anmeldung: **06.10.2021**
(21) Anmeldenummer: 20213976.2
(22) Anmeldetag: 15.12.2020
(51) Int. Cl.: A61K 9/00, A61K 31/216, A61M 5/315, A61M 5/178, A61P 13/10, A61M 5/31

(54) **SPRITZE UND KIT FÜR DIE INTRAVESIKALE ANWENDUNG**

(30) Priorität: 03.04.2020 DE 102020002136; 23.04.2020 DE 102020111080
(71) Anmelder: Farco-Pharma GmbH, 50670 Köln (DE)
(72) Erfinder: SODHA, Frank, 50670 Köln (DE); KÜHBACHER, Andreas, 50670 Köln (DE)
(74) Vertreter: Strehlke, Ingo Kurt

(57) **Zusammenfassung**

Die vorliegende Erfindung betrifft ein Verfahren zur Herstellung einer sterilen oxybutyninhaltigen Zusammensetzung in einer vorzugsweise anwendungsfertigen Kolbenspritze bzw. zur Herstellung einer vorzugsweise anwendungsfertigen, sterilen, mit einer oxybutyninhaltigen Zusammensetzung befüllten Kolbenspritze sowie auch eine sterile oxybutyninhaltige Zusammensetzung und Kolbenspritze als solche.

## Beschreibung

Die vorliegende Erfindung betrifft das medizinisch-technische Gebiet der Sterilisation bzw. der Keimreduzierung einer speziellen Zusammensetzung, welche Oxybutyninhydrochlorid als Wirkstoff aufweist und welche in eine insbesondere anwendungsfertige Kolbenspritze eingebracht ist bzw. vorliegt.

Insbesondere betrifft die vorliegende Erfindung ein Verfahren zur Herstellung einer sterilen oxybutyninhaltigen Zusammensetzung in einer vorzugsweise anwendungsfertigen Kolbenspritze bzw. zur Herstellung einer vorzugsweise anwendungsfertigen, sterilen, mit einer oxybutyninhaltigen Zusammensetzung befüllten Kolbenspritze.

Die vorliegende Erfindung betrifft auch eine sterile oxybutyninhaltige Zusammensetzung als solche, wie sie insbesondere zur Verwendung bei der prophylaktischen bzw. therapeutischen Behandlung von neurogenen Blasenfunktionsstörungen, insbesondere mittels Instillation in die Harnblase, eingesetzt wird.

Darüber hinaus betrifft die vorliegende Erfindung auch eine mit der sterilen oxbutyninhaltigen Zusammensetzung befüllte Kolbenspritze als solche, wobei die in der Kolbenspritze enthaltene oxybutyninhaltige Zusammensetzung einschließlich der Kolbenspritze nach dem erfindungsgemäßen Verfahren dampfsterilisiert worden ist.

Weiterhin betrifft die vorliegende Erfindung auch eine Verpackungseinheit, welche mindestens eine erfindungsgemäße Kolbenspritze aufweist, wobei die Kolbenspritze in eine Verpackung eingebracht ist bzw. in einer Verpackung vorliegt.

Weiterhin betrifft die vorliegende Erfindung auch entsprechende Kits auf Basis der erfindungsgemäßen Kolbenspritze bzw. der oxybutyninhaltigen Zusammensetzung nach der Erfindung.

Die vorliegende Erfindung betrifft auch die Verwendung einer Kolbenspritze in einem Verfahren zur Herstellung einer sterilen oxybutyninhaltigen Zusammensetzung sowie weiterhin auch die Verwendung einer Dampfsterilisation in einem Verfahren zur Herstellung einer sterilen oxybutyninhaltigen Zusammensetzung in einer vorzugsweise anwendungsfertigen Kolbenspritze.

Der Wirkstoff Oxybutynin (synonym auch als 4-Diethylaminobut-2-inyl-2-cyclohexyl-2-hydroxy-2-phenylethanoat bezeichnet) bzw. Oxybutyninhydrochlorid ist ein Anticholinergikum, welches zur Behandlung von Blasenfunktionsstörungen bzw. Dysfunktionen der Harnblase eingesetzt wird, und zwar insbesondere von solchen Blasenfunktionsstörungen, welche mit einer Detrusorhyperaktivität und/oder eine Detrusor-Sphinkter-Dyssynergie einhergehen bzw. hierdurch bedingt sind.

Neurogene Blasenfunktionsstörungen (nBFS) bzw. neurogene Blasenentleerungsstörungen stellen eine oftmals therapiebedürfte pathologische Situation der Harnblase und der an der Harnspeicherung und Harnausscheidung beteiligten anatomischen Strukturen dar. Bei neurogenen Blasenfunktionsstörungen handelt es sich insbesondere um solche Dysfunktionen der Harnblase bzw. der relevanten anatomischen Strukturen, die infolge einer Fehlfunktion oder Verletzung des Nervensystems auftreten, beispielsweise infolge von Rückenmarksverletzungen, Spina bifida ("offener Rücken"), Diabetes, Multiple Sklerose, Schlaganfall oder Morbus Parkinson. Der mit der Dysfunktion der Harnblase einhergehende Leidensdruck von Patienten ist dabei mitunter sehr groß, zumal die oftmals bereits schon eingeschränkte Lebensqualität durch die mit der Dysfunktion einhergehenden Beschwerden zusätzlich verschlechtert wird.

Im Allgemeinen wird als neurogene Blasenfunktionsstörung (nBFS) somit eine Störung der Harnspeicherung bzw. der Harnleerung bezeichnet, welche durch neurologische Veränderungen bzw. Verletzungen im Bereich des Rückenmarks im Bereich der für die Blasenfunktion relevanten Abschnitte des Gehirns oder im Bereich der Peripherie verursacht werden, wobei eine normale nervale Signalübertragung zur Steuerung der Harnblase oftmals beeinträchtigt oder behindert ist. Infolge der zugrundeliegenden neurologischen Schäden ist die natürliche Funktion der Harnblase im Hinblick auf deren Funktionszustände, nämlich einer (Harn-)Füllphase zum einen und einer (Harn-)Entleerungs- bzw. Miktionsphase zum anderen, gestört. Dabei kann der physiologische Ablauf von Blasenfüllung und Blasenentleerung sogar auf mehreren Ebenen beeinträchtigt bzw. unterbunden sein.

Infolge der neurologischen Fehlfunktion liegt bei einer neurogenen Blasenfunktionsstörung (nBFS) oftmals eine Detrusorhyperaktivität und/oder eine Detrusor-Sphinkter-Dyssynergie vor. Damit geht oftmals eine Störung des Zusammenspiels bzw. der Koordination der für die natürliche Blasenfunktion relevanten anatomischen Strukturen einher.

Der Detrusor (*Musculus detrusor vesicae* bzw. "Austreiber der Harnblase") stellt ein kräftiges Muskelsystem dar, welches die Harnblase umgibt bzw. einen Teil der Harnblase bildet, wobei es sich bei dem Detrusor um einen glatten Muskel handelt, welcher in einer physiologischer Situation bei der Miktion bzw. Blasenentleerung und somit der Harnaustreibung eine große Rolle spielt. Dabei wird durch Signale des Parasympathikus eine Anspannung des Muskelsystems hervorgerufen, wodurch der Blaseninhalt unter Druck gesetzt wird. Zudem wird der Detrusor durch einen intrinsischen Nervenplexus beeinflusst, welcher in der Blasenwand liegt und den Tonus an den Füllungszustand der Harnblase anpasst.

In der pathologischen Situation liegt bei einer neurogenen Blasenfunktionsstörung oftmals eine Detrusorhyperaktivität vor, welche in diesem Zusammenhang auch als neurogene Detrusorhyperaktivität (*Neurogenic Detrusor Overactifity, NDO*) bezeichnet wird. Eine Detrusorhyperaktivität, welche mit einer relevanten neurologischen Grunderkrankung in Verbindung steht, geht dabei insbesondere mit Pollakisurie, Nykturie sowie einer übermäßigen Drangsymptomatik einher.

Bei einer Detrusor-Sphinkter-Dyssynergie (DSD) ist zudem das Zusammenwirken der bei der Blasenentleerung beteiligten anatomischen Strukturen gestört, wobei eine Detrusorüberaktivität einer spastischen Funktionsstörung der Beckenbodenmuskulatur bzw. des äußeren Blasenschließmuskels gegenübersteht, was zu einer Obstruktion des Blasenausgangs bei gleichzeitiger Miktionsanstrengung führt. Typisch für eine Detrusor-Sphinkter-Dyssynergie ist dabei ein häufig unterbrochener Harnstrahl und Schwierigkeiten bei der Initiierung der Miktion. Darüber hinaus liegen oftmals auch Pollakisurie sowie eine Restharnbildung vor.

Neben den vorgenannten Symptomen ist eine neurogene Blasenfunktionsstörung oftmals auch mit hohen intravesikalen Drücken verbunden, d. h. aufgrund der Hyperaktivität des Detrusor bzw. der Detrusor-Sphinkter-Dyssynergie wird ein übermäßiger Druck in der Harnblase aufgebaut. Dies stellt nicht zuletzt auch eine Gefahr für den oberen Harntrakt dar, so gehen übermäßig hohe intravesikale Drücke oftmals mit einer Nierenschädigung einher bzw. rufen diese hervor.

Im Lichte der obigen Ausführungen besteht somit im Stand der Technik ein großer Bedarf an der Bereitstellung therapeutischer Konzepte, welche bei Vorliegen einer neurogenen Blasenfunktionsstörung zu einer Normalisierung bzw. Verbesserung der Blasenfunktion bzw. zu einer Verringerung der Symptome führen, und zwar sowohl was die Probleme beim Harnlassen als auch die vorliegenden hohen Drücke in der Harnblase anbelangt. Dabei besteht ein wesentliches Ziel neben der Verbesserung der Miktion auch in der Verringerung des intrinsischen Drucks, um hierdurch etwaige Nierenschäden oder dergleichen zu vermeiden.

In diesem Zusammenhang verfolgt die Therapie der neurogenen Blasenfunktionsstörung somit mehrere Ziele, und zwar insbesondere den Schutz des oberen Harntraktes und der Nierenfunktion, die Verbesserung der Kontinenzsituation, die Wiederherstellung bzw. Verbesserung der Funktion des unteren Harntraktes sowie nicht zuletzt die Steigerung der Lebensqualität.

Neben konservativen Maßnahmen, wie regelmäßige Bewegung, gezieltes Blasentraining oder dergleichen, kommen auch pharmakotherapeutische Maßnahmen zur Behandlung neurogener Blasenfunktionsstörungen zum Einsatz. Beispielsweise kann auf Basis minimalinvasiver Verfahren eine Injektion mit Botulinumtoxin in die Harnblasenwand durchgeführt werden, um hierdurch die Aktivität des Detrusors zu vermindern. Die Verabreichung ist jedoch relativ aufwendig und nur durch einen Arzt durchzuführen, da die Wirksubstanz relativ aufwendig in die Blasenwandung injiziert werden muss. Weiterhin kommen auch minimalinvasive Verfahren in Form von sakraler Neuromodulation zum Einsatz, bei welcher im oberen Gesäßbereich ein Schrittmacher implantiert wird, welcher über eine Elektrode schwache elektrische Impulse an die Sakralnerven abgibt. Der diesbezügliche Einsatzbereich ist jedoch im Hinblick auf die zugrundeliegenden Grunderkrankungen beschränkt. Zudem handelt es sich hierbei um einen nicht unwesentlichen operativen Eingriff, welcher mit weiteren Risiken einhergeht.

Zudem sind im Stand der Technik pharmakologische Therapien bekannt, bei denen auch Anticholinergika zum Einsatz kommen. In diesem Zusammenhang wird insbesondere auf eine Verabreichung von Oxybutynin bzw. Oxybutyninhydrochlorid, Trospiumchlorid, Propiverin, Tolterodin oder dergleichen abgestellt. Dabei besteht im Stand der Technik ein Fokus auf der systemischen bzw. oralen Applikation bzw. Verabreichung dieser Wirksubstanzen. Die systemische Verabreichung kann jedoch im verstärkten Maße mit systemischen Nebenwirkungen einhergehen, wie Restharnbildung, Obstipation, Akkomodationsstörungen, Mundtrockenheit, Tachykadie, Herzrhythmusstörungen und dergleichen. Darüber hinaus ist die Konzentration der Wirksubstanz am Wirkort, nämlich der Harnblase, mitunter nicht optimal einzustellen.

Darüber hinaus ist eine systemische Verabreichung der Wirksubstanz in Form von Anticholinergika insofern nachteilig, als ein Teil der verabreichten Wirksubstanz bei deren erster Passage im Gastrointestinalbereich bzw. in der Leber verstoffwechselt wird (sogenannter *First pass*-Metabolismus), wobei mitunter nichtwirksame Metaboliten entstehen, so dass die Bioverfügbarkeit am Wirkort geringer ausfällt bzw. die Wirkmenge am Wirkort nur schwer einzustellen bzw. vorzugeben ist. Folglich können Patienten mit systemischen Darreichungsformen mitunter nicht hinreichend eingestellt werden.

Die WO 2012/154779 A1 bzw. die zu derselben Patentfamilie gehörende US 2012/0289564 A1 betrifft die Verwendung einer Kombination von Oxybutynin und einer die Bildung von speichelanregenden Substanz zur Behandlung der überaktiven Blase, wobei diesbezüglich auf eine orale Applikation fokussiert wird.

Darüber hinaus können Anticholinergika topisch appliziert werden, beispielsweise mittels eines transdermalen Pflasters oder dergleichen. Auch bei dieser systemischen Applikation können im erhöhten Maße systemische Nebenwirkungen auftreten. Zudem besteht auch hier das Problem einer vorzeitigen Verstoffwechselung.

Darüber hinaus können Anticholinergika, wie Oxybutynin bzw. Oxybutyninhydrochlorid, topisch in den Urogenitalbereich, insbesondere in die Harnblase, appliziert bzw. instilliert werden, beispielsweise über einen Katheter oder dergleichen. Durch die diesbezüglich lokale Applikation bzw. die diesbezüglich vorgesehene Instillation erfolgt eine Darreichung der Wirksubstanz unmittelbar an seinem Wirkort, wodurch die gezielte Wirkung bzw. die Bioverfügbarkeit erhöht und systemische Nebenwirkungen reduziert werden. Zudem wird der Wirkstoff bei dieser Verabreichungsart nicht vorzeitig über andere Organe verstoffwechselt. Zudem kann eine diesbezügliche Verabreichung gegebenenfalls auch von dem Patienten selbst vorgenommen werden.

Oxybutynin bzw. Oxybutyninhydrochlorid wirkt dabei insbesondere als kompetitiver Antagonist von Acetylcholin auf postganionäre Muskarinrezeptoren, was zu einer Entspannung bzw. krampflösenden Wirkung im Hinblick auf die glatte Blasenmuskulatur und somit auch zu einer Abnahme der Detrusorhyperaktivität führt. Insbesondere wird die Blasenkontraktion gehemmt, wobei auch Spasmen gelockert werden. Zudem führt die Verabreichung von Oxybutynin bzw. Oxybutyninhydrochlorid zu einer Vergrößerung des Blasenvolumens und zu einer Verringerung von Kontraktionen, wobei auch der Drang zum Harnlassen bei neurogenen Blasenfunktionsstörungen verringert bzw. verzögert wird. Weiterhin kann durch den Einsatz von Oxybutynin bzw. Oxybutyninhydrochlorid auch der intravesikale (Blasen-)Druck verringert werden, was auch zu einer Entlastung der oberen Harnwege führt. Darüber hinaus weist Oxybutynin bzw. Oxybutyninhydrochlorid Eigenschaften eines Lokalanästhetikums auf. Oxybutynin bzw. Oxybutyninhydrochlorid gehört dabei insbesondere zur Gruppe der Parasymphatolytika.

Im Rahmen der im Stand der Technik vorgesehenen Instillation bzw. topischen Applikation in die Harnblase wird Oxybutynin bzw. Oxybutyninhydrochlorid im Allgemeinen in Form einer wässrigen Zusammensetzung bzw. wässrigen Lösung eingesetzt, wobei die wirkstoffhaltige Zusammensetzung bzw. Lösung unter Einsatz entsprechender Applikationsvorrichtungen in die Harnblase appliziert bzw. verabreicht wird.

Dabei kommt der diesbezüglichen Sterilität einer für die Instillation bzw. topischen Verabreichung in die Harnblase eingesetzten Zusammensetzung eine hohe Bedeutung zu, und zwar insbesondere vor dem Hintergrund etwaige Infektionen der hierfür besonders anfälligen und mitunter durch das Krankheitsbild bereits vorgeschädigten Harnblase zu vermeiden.

Um eine Verkeimung zu vermeiden, können die wirkstoffhaltigen Zusammensetzungen beispielsweise unmittelbar vor deren Anwendung bzw. Verabreichung hergestellt werden, was aber aufwendig ist und zudem noch mit einer gewissen Gefahr der Kontamination der Zusammensetzung bei deren Herstellung einhergeht. Weiterhin können die wirkstoffhaltigen Zusammensetzungen als solche sterilisiert werden, beispielsweise mittels Mikrofiltrationsverfahren oder dergleichen, und anschließend in eine Aufbewahrungs- bzw. Applikationsvorrichtung abgefüllt werden. Auch hierbei kann es jedoch zu einer unerwünschten Kontaminationen kommen, insbesondere im Hinblick auf mögliche an der Aufbewahrungs- bzw. Verabreichungsvorrichtung anhaftende Keime oder dergleichen. In diesem Zusammenhang kann auch eine separate Sterilisation der Aufbewahrungs- bzw. Applikationsvorrichtung vorgesehen sein, was aber gleichermaßen zu mitunter nicht zufriedenstellenden Sterilisationsergebnissen führt, insbesondere bedingt durch das nachträgliche Abfüllen der Zusammensetzung in die Aufnahme- bzw. Applikationsvorrichtung. In diesem Zusammenhang kann zudem unter sterilen Bedingungen, beispielsweise unter Reinraumbedingungen, gearbeitet werden, was aber mit einem gewissen Aufwand einhergeht und zudem kostenintensiv ist.

Problematisch bei einer Sterilisation ist darüber hinaus auch, dass die zu sterilisierende wirkstoffhaltige Zusammensetzung mitunter nicht die erforderliche Stabilität unter den notwendigen bzw. angewandten Sterilisationsbedingungen aufweist, so dass es beispielsweise zu einem übermäßigen Abbau der Wirksubstanz, einhergehend mit der Bildung unerwünschter Abbauprodukte, kommen kann, was sowohl die Wirksamkeit als auch die Anwendungssicherheit einer derartigen Zusammensetzung negativ beeinflusst. So liegt auch für Zusammensetzungen unter Verwendung von Oxybutynin bzw. Oxybutyninhydrochlorid als Wirksubstanz eine gewisse Instabilität vor, so dass eine diesbezügliche Sterilisierung zu einer Verringerung der Qualität der Zusammensetzung führen kann.

Insgesamt besteht somit im Stand der Technik ein großer Bedarf an entsprechenden Konzepten, die zu einer optimalen Sterilisierung von in entsprechenden Aufnahme- bzw. Applikationsvorrichtungen vorliegenden Zusammensetzungen mit Oxybutynin bzw. Oxybutyninhydrochlorid als Wirksubstanz führen, und dies unter Beibehaltung der Stabilität der Zusammensetzungen, um auf dieser Basis zu anwendungsfertigen Produkten zu gelangen, welche im Hinblick auf die Instillation in die Harnblase bei hoher Wirksamkeit entsprechend anwendungssicher sind (d. h. bei hohem Wirkstoffgehalt bzw. geringer Menge an Abbauprodukten nicht zu Infektionen führen). Dabei soll auch eine hohe (Lager-)Stabilität der Zusammensetzung gegeben sein.

Vor diesem Hintergrund besteht eine Aufgabe der vorliegenden Erfindung darin, ein effizientes Verfahren zur Herstellung einer sterilen Zusammensetzung, welche Oxybutynin bzw. Oxybutyninhydrochlorid als Wirkstoff aufweist und welche in einer vorzugsweise anwendungsfertigen Aufbewahrungs- bzw. Applikationsvorrichtung, nämlich einer Kolbenspritze, vorliegt, bereitzustellen, wobei die zuvor geschilderten Nachteile des Standes der Technik zumindest weitgehend vermieden oder aber wenigstens abgeschwächt werden sollen.

Insbesondere ist eine Aufgabe der vorliegenden Erfindung darin zu sehen, ein diesbezügliches Verfahren bereitzustellen, welches zu einer anwendungsfertigen Aufbewahrungs- bzw. Applikationsvorrichtung führt, welche eine entsprechende Zusammensetzung mit Oxybutynin bzw. Oxybutyninhydrochlorid als Wirksubstanzen in steriler Form enthält, welche auch nach durchgeführter Sterilisation neben einer hohen Keimfreiheit auch einen hohen bzw. unveränderten Wirkstoffgehalt bzw. allenfalls einen geringen Gehalt an entsprechenden Abbauprodukten aufweist.

Diesbezüglich soll im Rahmen der vorliegenden Erfindung insbesondere ein solches Verfahren bereitgestellt werden, anhand dessen eine entsprechend keimfreie Zusammensetzung mit Oxybutynin bzw. Oxybutyninhydrochlorid als Wirksubstanz bereitgestellt wird, welche bei hoher Keimfreiheit bzw. Sterilität auch eine hohe Wirksamkeit und Verträglichkeit aufweist. Dabei sollen auch eine verbesserte Anwendungssicherheit und eine verbesserte (Lager-)Stabilität bzw. Haltbarkeit vorliegen. Insbesondere soll auf dieser Basis ein anwendungsfertiges Produkt bereitgestellt werden, bei welchem die entsprechende Zusammensetzung in einer diesbezüglichen Aufbewahrungs- bzw. Applikationsvorrichtung eingebracht vorliegt, und zwar in einer diesbezüglichen (Einweg-)Spritze bzw. Kolbenspritze.

Insbesondere soll im Rahmen einer weiteren Aufgabe der vorliegenden Erfindung auch eine mit der wirkstoffhaltigen Zusammensetzung befüllte Aufbewahrungs- bzw. Applikationsvorrichtung, und zwar in Form einer (Einweg-)Spritze bzw. Kolbenspritze, bereitgestellt werden, welche in verbesserter Weise einem Sterilisationsverfahren zugänglich ist, wobei eine effektive Sterilisation unter Beibehaltung der Stabilität des Befüllungsgutes in Form der wirkstoffhaltigen Zusammensetzung gewährleistet sein soll.

Zur Lösung der zuvor geschilderten Aufgabe schlägt die vorliegende Erfindung somit - gemäß einem **ersten** Aspekt der vorliegenden Erfindung - ein Verfahren zur Herstellung einer sterilen oxybutyninhaltigen Zusammensetzung in einer vorzugsweise anwendungsfertigen Kolbenspritze gemäß Patentanspruch 1 vor; jeweils vorteilhafte Weiterbildungen und Ausgestaltungen dieses Erfindungsaspekts sind Gegenstand der das Verfahren betreffenden Unter- bzw. Nebenansprüche.

Weiterer Gegenstand der vorliegenden Erfindung - gemäß einem **zweiten** Aspekt der vorliegenden Erfindung - ist zudem die sterile oxybutyninhaltige Zusammensetzung, wie sie insbesondere zur Verwendung bei der prophylaktischen bzw. therapeutischen Behandlung von neurogenen Blasenfunktionsstörungen eingesetzt werden soll, gemäß den diesbezüglichen, die erfindungsgemäße Zusammensetzung betreffenden unabhängigen Patentansprüchen; jeweils vorteilhafte Weiterbildungen und Ausgestaltungen dieses Erfindungsaspektes sind Gegenstand der die Zusammensetzung nach der Erfindung betreffenden Unteransprüche.

Wiederum weiterer Gegenstand der vorliegenden Erfindung - gemäß einem **dritten** Aspekt der vorliegenden Erfindung - ist auch die erfindungsgemäße Kolbenspritze, welche die sterile oxybutyninhaltige Zusammensetzung nach der Erfindung aufweist, gemäß den diesbezüglichen, die erfindungsgemäße Kolbenspritze betreffenden unabhängigen Patentansprüchen; jeweils vorteilhafte Weiterbildungen und Ausgestaltungen dieses Erfindungsaspekts sind Gegenstand der die erfindungsgemäße Kolbenspritze betreffenden Unteransprüche.

Nochmals weiterer Gegenstand der vorliegenden Erfindung - gemäß einem **vierten** Aspekt der vorliegenden Erfindung - ist auch die erfindungsgemäße Verpackungseinheit, welche die erfindungsgemäße Kolbenspritze mit der diesbezüglichen Zusammensetzung enthält, gemäß dem diesbezüglichen, die Verpackungseinheit nach der Erfindung betreffenden unabhängigen Patentanspruch.

Gegenstand der vorliegenden Erfindung sind weiterhin - gemäß einem **fünf**te**n** Aspekt der vorliegenden Erfindung - auch die erfindungsgemäße Kits (*kit-of-parts*), gemäß dem diesbezüglichen, die erfindungsgemäßen Kits betreffenden unabhängigen Patentanspruch.

Wiederum weiterer Gegenstand der vorliegenden Erfindung - gemäß einem **sechsten** Aspekt der vorliegenden Erfindung - ist auch die Verwendung einer speziellen Kolbenspritze in einem Verfahren zur Herstellung einer sterilen oxybutyninhaltigen Zusammensetzung gemäß dem diesbezüglichen, die erfindungsgemäße Verwendung betreffenden unabhängigen Patentanspruch.

Schließlich ist Gegenstand der vorliegenden Erfindung - gemäß einem **siebten** Aspekt der vorliegenden Erfindung - die erfindungsgemäße Verwendung einer Dampfsterilisation zur Bereitstellung einer sterilen oxybutyninhaltigen Zusammensetzung gemäß dem diesbezüglichen diese Verwendung betreffenden unabhängigen Patentanspruch.

Es versteht sich von selbst, dass Ausgestaltungen, Ausführungsformen, Vorteile und dergleichen, welche nachfolgend zu Zwecken der Vermeidung von Wiederholungen zur zu einem Erfindungsaspekt aufgeführt sind, selbstverständlich auch in Bezug auf die übrigen Erfindungsaspekte entsprechend gelten, ohne dass dies einer gesonderten Erwähnung bedarf.

Darüber hinaus verhält es sich im Hinblick auf die nachfolgende Beschreibung der vorliegenden Erfindung auch derart, dass die jeweils im Zusammenhang mit den speziellen Ausgestaltungen, Ausführungsformen, Vorteilen, Beispielen oder dergleichen angeführten Merkmale der vorliegenden Erfindung auch in deren Kombination als offenbart gelten. Somit gelten vorliegend auch übergeordnete Kombinationen einzelner oder mehrerer Merkmale, welche für jeweilige Ausgestaltungen, Ausführungsformen, Anwendungsbeispiele oder dergleichen angeführt sind, als offenbart.

Bei allen nachstehend genannten relativen bzw. prozentualen gewichtsbezogenen Angaben, insbesondere Mengen- bzw. Konzentrationsangaben, ist weiterhin zu beachten, dass diese im Rahmen der vorliegenden Erfindung vom Fachmann derart auszuwählen sind, dass sie sich in der Summe unter Einbeziehung aller Komponenten bzw. Inhaltsstoffe, insbesondere wie nachfolgend definiert, zu 100 % bzw. 100 Gew.-% ergänzen bzw. addieren; dies versteht sich aber für den Fachmann von selbst.

Weiterhin gilt, dass der Fachmann - anwendungsbezogen oder einzelfallbedingt - von den nachfolgend angeführten Konzentrations-, Gewichts-, Mengen- und Bereichsangaben abweichen kann, ohne dass er den Rahmen der vorliegenden Erfindung verlässt.

Zudem gilt, dass alle im Folgenden genannten Werte- bzw. Parameterangaben oder dergleichen grundsätzlich mit genormten bzw. standardisierten oder explizit angegebenen Bestimmungsverfahren und andernfalls mit dem Fachmann auf diesem Gebiet an sich geläufigen Bestimmungs- bzw. Messmethoden ermittelt bzw. bestimmt werden können. Sofern nicht anders angegeben, werden die zugrundeliegenden Werte bzw. Parameter unter Standardbedingungen (d. h. insbesondere bei einer Temperatur von 20 °C und/oder bei einem Druck von 1.013,25 hPa bzw. 1,01325 bar) ermittelt.

Zu Zwecken der Veranschaulichung der vorliegenden Erfindung wird in der nachfolgenden Beschreibung der erfindungsgemäßen Gegenstände auch auf die in der Figur angeführten Bezugszeichen zurückgegriffen; die diesbezügliche Anführung der Bezugszeichen ist dabei rein veranschaulichend und geht mit keinerlei Beschränkung der erfindungsgemäßen Gegenstände einher.

Dies vorausgeschickt wird im Folgenden die vorliegende Erfindung im Detail erläutert:
Gegenstand der vorliegenden Erfindung - gemäß einem **ersten** Aspekt der vorliegenden Erfindung - ist somit ein Verfahren zur Herstellung einer sterilen oxybutyninhaltigen Zusammensetzung in einer vorzugsweise anwendungsfertigen Kolbenspritze, insbesondere Einweg-Kolbenspritze, und/oder zur Herstellung einer vorzugsweise anwendungsfertigen, sterilen, mit einer oxybutyninhaltigen Zusammensetzung befüllten Kolbenspritze, insbesondere Einweg-Kolbenspritze,
wobei die Kolbenspritze einen Spritzenkörper, insbesondere Spritzenzylinder, einerseits und einen Spritzenkolben mit einem Kolbenstopfen andererseits aufweist, wobei der Spritzenkörper aus einem Cycloolefin-(Co-)Polymer (COP bzw. COC; mit COP = Cycloolefin-Polymer und COC = Cycloolefin-Copolymer) gebildet ist und wobei der Kolbenstopfen aus einem Halogenbutylkautschuk gebildet ist;
wobei das Verfahren die nachfolgenden Schritte umfasst:
   a) Befüllen der Kolbenspritze mit der oxybutyninhaltigen Zusammensetzung und/oder Bereitstellen der oxybutyninhaltigen Zusammensetzung in der Kolbenspritze,
      wobei das Oxybutynin in Form des Hydrochlorids eingesetzt wird, die oxybutyninhaltige Zusammensetzung als wässrige Zusammensetzung ausgebildet wird und die oxybutyninhaltige Zusammensetzung in Kombination und jeweils in wirksamen, insbesondere pharmazeutisch wirksamen Mengen,
      - Oxybutyninhydrochlorid (Oxybutynin-HCI) in einer Konzentration von (1 ± 0,5) mg/ml und
      - Natriumchlorid, berechnet als Natrium, in einer Konzentration von (3,5 ± 0,5) mg/ml
      enthält,
      wobei die oxybutyninhaltige Zusammensetzung auf einen pH-Wert im Bereich von 3 bis 5, insbesondere im Bereich von 3,8 bis 4,5, eingestellt wird und/oder wobei die oxybutyninhaltige Zusammensetzung einen pH-Wert im Bereich von 3 bis 5, insbesondere im Bereich von 3,8 bis 4,5, aufweist;
      und nachfolgend
   b) Dampfsterilisation, insbesondere Wasserdampfsterilisation, der oxybutyninhaltigen Zusammensetzung in der Kolbenspritze und/oder der mit der oxybutyninhaltigen Zusammensetzung befüllten Kolbenspritze.

In diesem Zusammenhang hat die Anmelderin in völlig überraschender Weise gefunden, dass bei Verwendung einer speziellen Sterilisation, nämlich einer Dampfsterilisation, eine speziell ausgebildete oxybutyninhaltige Zusammensetzung unter Einsatz einer wiederum sehr speziellen Kolbenspritze in effektiver Weise sterilisiert werden kann, wobei in diesem Zusammenhang im Rahmen des erfindungsgemäßen Verfahrens auch eine hohe Stabilität der Zusammensetzung bei Durchführung der Dampfsterilisation gewährleistet ist. So verhält es sich für die im Rahmen des erfindungsgemäßen Verfahrens dampfsterilisierte oxybutyninhaltige Zusammensetzung nämlich derart, dass diese auch nach erfolgter Sterilisation einen hohen Wirkstoffgehalt aufweist, da die Wirksubstanz Oxybutyninhydrochlorid während der Dampfsterilisation allenfalls nur in geringen Mengen abgebaut wird (was mit der Bildung von allenfalls nur geringen Mengen an Abbauprodukten einhergeht). Somit resultiert auf Basis des erfindungsgemäßen Verfahrens eine sterile Zusammensetzung mit hoher Anwendungssicherheit und Wirkeffizienz. Insbesondere liegt auch nach erfolgter Dampfsterilisation eine entsprechend hohe Wirksamkeit bzw. Wirkeffizienz der diesbezüglich sterilisierten oxybutyninhaltigen Zusammensetzung vor.

Im Hinblick auf das erfindungsgemäße Verfahren kommt auch der diesbezüglichen Abstimmung der eingesetzten Komponenten in Form der oxybutyninhaltigen Zusammensetzung einerseits und der verwendeten Kolbenspritze andererseits eine große Bedeutung zu. So verhält es sich im Hinblick auf die sehr speziell ausgebildete oxybutyninhaltige Zusammensetzung, wie sie im Rahmen des erfindungsgemäßen Verfahrens eingesetzt wird derart, dass diese eine hohe Kompatibilität zu der Kolbenspritze aus Cycloolefin-(Co-)Polymer (COP bzw. COC) und dem Kolbenstopfen aus Halogenbutylkautschuk aufweist, was zu der hohen Stabilität der Zusammensetzung bei der speziell eingesetzten Dampfsterilisation führt. Folglich kann infolge dieser speziellen Abstimmung der Abbau an Wirksubstanz bzw. die Ausbildung von Abbauprodukten deutlich vermindert bzw. verringert werden.

Somit kommt im Hinblick auf das erfindungsgemäße Verfahren der gezielten Kombination der zuvor angeführten technischen Maßnahmen, nämlich Bereitstellung einer sehr speziellen oxybutyninhaltigen Zusammensetzung mit definierten Mengen an diesbezüglich eingesetzten Komponenten und definiertem pH-Wert sowie Auswahl spezieller Materialien für die Komponenten der eingesetzten Kolbenspritze, im Hinblick auf die durchgeführte Dampfsterilisation eine zentrale Bedeutung zu, da infolge der diesbezüglich speziellen Auswahl und Abstimmung eine hohe Kompatibilität bei der Dampfsterilisation vorliegt.

Demgegenüber konnte die Anmelderin jedoch auch zeigen, dass für Oxybutynin bzw. Oxybutyninhydrochlorid in wässrigen Zusammensetzungen bei thermischer Sterilisation - im Gegensatz zur vorliegenden Erfindung - eine hohe Instabilität bzw. Inkompatibilität vorliegt, und zwar für den Fall, dass im Rahmen einer Sterilisation (Einweg-)Spritzen auf Basis von Polypropylen und Kolbenstopfen bzw. Gummistopfen sowie Verschlusskappen aus üblicherweise eingesetzten Gummimaterialien verwendet werden. Denn hierbei kommt es im Gegensatz zur vorliegenden Erfindung bei einer Sterilisation zu einem erhöhten Abbau der Wirksubstanz, einhergehend mit der erhöhten Bildung von unerwünschten Abbauprodukten, so dass die resultierende Produktqualität bzw. Anwendungssicherheit sowie Wirksamkeit für diesen Fall insgesamt verschlechtert ist. In diesem Zusammenhang konnte auch gezeigt werden, dass für Zusammensetzungen, welche Oxybutyninhydrochlorid als Wirksubstanz aufweisen, bei der Sterilisation unter Verwendung von (Einweg-)Spritzen auf Basis von Polypropylen als Spritzenmaterial und Kolbenstopfen sowie Verschlusskappen aus diesbezüglich üblicherweise eingesetzten Gummimaterialien das Abbauprodukt Phenylcyclohexylglycolsäure entsteht, so dass die Produktqualität nachhaltig beeinflusst ist. Dies konnte im Rahmen der vorliegenden Erfindung nunmehr nachhaltig überkommen werden, wie vorliegend dargelegt.

Im Rahmen der vorliegenden Erfindung ist es somit gelungen, die zuvor aufgezeigten Inkompatibilitäten zu überkommen, und dies bei gleichzeitig hoher Sicherheit und Effizienz im Hinblick auf die Bereitstellung entsprechend steriler Zusammensetzungen.

In diesem Zusammenhang ist es gleichermaßen völlig überraschend, dass die gezielte Auswahl der für die erfindungsgemäß eingesetzten Kolbenspritze, wonach nämlich zumindest der Spritzenkörper aus einem Cycloolefin-(Co-)Polymer (COP bzw. COC) gebildet ist, und wonach der Kolbenstopfen der Spritze in spezieller Weise aus eine Halogenbutylkautschuk (und zwar besonders bevorzugt aus Brombutylkautschuk) gebildet ist, zu einer erhöhten Stabilität der zu sterilisierenden Zusammensetzung bei deren Dampfsterilisation führt, einhergehend mit entsprechend verringerten Mengen an unerwünschten Abbauprodukten bei gleichzeitig hohem Wirkstoffgehalt der sterilisierten Zusammensetzung. Die diesbezüglichen Eigenschaften werden auch dadurch weiter verbessert bzw. gewährleistet, dass eine spezielle oxybutyninhaltige Zusammensetzung eingesetzt wird, welche nämlich über definierte Konzentrationen an Oxybutyninhydrochlorid und an Natriumchlorid verfügt, wobei zudem ein spezieller pH-Wert eingestellt ist. Erfindungsgemäß wird auf dieser Basis somit insgesamt im Rahmen der herangezogenen Dampfsterilisation eine hohe Kompatibilität der insgesamt zu sterilisierenden Komponenten gewährleistet.

Im Rahmen der vorliegenden Erfindung wird hinsichtlich der Sterilisation der oxybutyninhaltigen Zusammensetzung in ziel- und zweckgerichteter Weise auf eine Dampfsterilisation abgestellt, anhand derer erfindungsgemäß hervorragende Sterilisationsergebnisse bei gleichzeitig hoher Produktstabilität erhalten werden.

Das Verfahren der Dampfsterilisation zählt dabei zu den thermischen Sterilisationsverfahren, wobei durch den Einsatz von (Wasser-)Dampf auch eine gute Energie- bzw. Wärmeübertragung auf die zu sterilisierenden Komponenten gewährleistet wird. Insbesondere wird bei der Kondensation des Dampfes an dem kühleren Sterilisationsgut die Wärmeenergie bzw. -menge auf die zu sterilisierende Kolbenspritze bzw. die zu sterilisierende oxybutyninhaltige Zusammensetzung übertragen, wodurch Mikroorganismen oder dergleichen abgetötet bzw. irreversibel inaktiviert werden. Im Rahmen der erfindungsgemäß eingesetzten Dampfsterilisation wird somit eine sozusagen vollständige bzw. gemeinsame und zeitgleiche Sterilisation der eingesetzten Kolbenspritze mit der darin befindlichen oxybutyninhaltigen Zusammensetzung gewährleistet, worin ein weiterer Vorteil beispielsweise auch gegenüber einer sterilen Abfüllung oder dergleichen zu sehen ist, da hierdurch die Sterilisationssicherheit erhöht ist.

Im Rahmen der vorliegenden Erfindung wird somit insbesondere derart vorgegangen, dass eine mit der oxybutyninhaltigen Zusammensetzung vorgefüllte Spritze aus einem speziellen Material, wie zuvor definiert, vorzugsweise terminal dampfsterilisiert wird. Dies führt zu einer hohen Sterilisationssicherheit und -effizienz.

Im Allgemeinen wird unter dem Begriff der (Dampf-)Sterilisation, wie er im Rahmen der vorliegenden Erfindung verwendet wird, insbesondere eine Abtötung bzw. eine irreversible Inaktivierung von Mikroorganismen, Keimen, Viren oder dergleichen, welche sich an bzw. in einem zu sterilisierenden Objekt befinden, einschließlich ihrer Ruhestadien bzw. Überdauerungsformen, wie beispielsweise Endosporen oder dergleichen, verstanden. Da die vollständige Beseitigung bzw. Inaktivierung sämtlicher Erreger an bzw. in einem Gegenstand bzw. Objekt rein statistisch nicht mit vollständiger Sicherheit gewährleistet werden kann, gilt ein Gegenstand bzw. ein Objekt eines Sterilisierguts im Allgemeinen dann als steril, wenn die Wahrscheinlichkeit einer Kontamination mit vermehrungsfähigen Erregern einen bestimmten Wert nicht überschreitet. Diesbezüglich kann insbesondere ein Wert von höchstens 1 : 10⁶ bzw. 10⁻⁶ angeführt werden. Dies bedeutet, dass unter 1 Million Einheiten eines Sterilisierguts höchstens eine Einheit nicht steril ist (beispielsweise wenn diese Einheit mit einer koloniebildenden Einheit (KBE) eines Mikroorganismus bzw. Erregers kontaminiert ist bzw. der Restgehalt an Erregern je Einheit eines Sterilisierguts höchstens 10⁻⁶ KBE beträgt, was dazu führt, dass die Wahrscheinlichkeit für eine nicht sterile Einheit bei 1 : 10⁶ liegt). Ein Gegenstand kann demnach insbesondere dann als steril betrachtet werden, wenn der theoretische Wert von nicht mehr als einem lebenden Mikroorganismus in 10⁶ (1 Million) sterilisierten Einheiten des Endprodukts vorhanden ist. Diesbezüglich wird im Allgemeinen auch von dem *Sterility Assurance Level* (*SAL*) gesprochen. Hierzu kann auch auf die nachfolgenden Ausführungen verwiesen werden.

Bei dem erfindungsgemäß für die Kolbenspritze bzw. den Spritzenkörper eingesetzten Material in Form von Cycloolefin-(Co-)Polymer (COP bzw. COC) handelt es sich um eine Klasse von technischen Polymeren, welche beispielsweise durch metallocenkatalysierte Copolymerisation von Cycloolefinen mit Alk-1-enen gewonnen werden können. Im Allgemeinen sind Cycloolefin-(Co-)Polymere amorph und damit transparent, bezogen auf das sichtbare Spektrum des Lichts. Cycloolefin-(Co-)Polymere, wie sie erfindungsgemäß eingesetzt werden, weisen im Allgemeinen eine hohe Steifigkeit, Festigkeit und Härte sowie eine niedrige Dichte und hohe Transparenz auf, wobei zudem eine hohe Säurebeständigkeit vorliegt. Darüber hinaus weisen die in Rede stehenden Polymere auch eine hohe Biokompatibilität auf.

Wie zuvor angeführt, wird durch die Verwendung der in Rede stehenden Polymere eine hohe Kompatibilität im Rahmen der erfindungsgemäß eingesetzten Dampfsterilisation gewährleistet, was zu entsprechend verbesserten Eigenschaften der sterilisierten oxybutyninhaltigen Zusammensetzung führt. Im Hinblick auf die erfindungsgemäß eingesetzten Cycloolefin-(Co-)Polymere (COP bzw. COC) können markt- bzw. handelsübliche Produkte verwendet werden (wie beispielsweise TopPAcPolymer Topas COC Grade 6013B-51; Grade EuP 3.2.8).

Was weiterhin das erfindungsgemäß für den Kolbenstopfen sowie für die nachfolgend noch beschriebene Verschlussvorrichtung (Verschlusskappe) der Kolbenspritze eingesetzte Material in Form von Halogenbutylkautschuk (und zwar besonders bevorzugt in Form von Brombutylkautschuk) anbelangt, so hat die Anmelderin gefunden, dass diesbezüglich insbesondere im Rahmen der Dampfsterilisation eine nur geringere chemische Reaktivität bzw. eine nur geringe Aktivität gegenüber Oxybutyninhydrochlorid vorliegt. Insbesondere weist Halogenbutylkautschuk, insbesondere Brombutylkautschuk, eine nur geringere Anzahl an C-C-Doppelbindungen auf, so dass Halogenbutylkautschuk, insbesondere Brombutylkautschuk, weniger reaktiv ist als üblicherweise eingesetzte Gummimaterialien. Zudem weist Halogenbutylkautschuk, insbesondere Brombutylkautschuk, wie er im Rahmen der vorliegenden Erfindung eingesetzt wird, weitere Vorteile auf, und zwar insbesondere was dessen hohe Hitzestabilität, verbesserte Alterungseigenschaften und geringe Gaspermeabilität anbelangt. Diesbezüglich können markt- bzw. handelsübliche Produkte eingesetzt werden (wie z. B. Dätwyler V9340, FM257/2 Bromobutyl-Compound).

Erfindungsgemäß wird somit auf Basis der gezielten Kombination der Gesamtheit an erfindungsgemäßen Maßnahmen ein effizientes Verfahren zur Bereitstellung einer stabilen sowie sterilen oxybutyninhaltigen Zusammensetzung bereitgestellt.

Im Rahmen der vorliegenden Erfindung kann es insbesondere vorgesehen sein, dass die oxybutyninhaltige Zusammensetzung das Oxybutyninhydrochlorid (Oxybutynin-HCI) in einer Konzentration von (1 ± 0,4) mg/ml, insbesondere in einer Konzentration von (1 ± 0,3) mg/ml, vorzugsweise in einer Konzentration von (1 ± 0,2) mg/ml, bevorzugt in einer Konzentration von (1 ± 0,1) mg/ml, besonders bevorzugt in einer Konzentration von etwa 1 mg/ml, enthält. Insbesondere kann die oxybutyninhaltige Zusammensetzung das Oxybutyninhydrochlorid in einer Konzentration von 1 mg /ml enthalten.

Weiterhin kann die oxybutyninhaltige Zusammensetzung das Natriumchlorid, berechnet als Natrium, in einer Konzentration von (3,5 ± 0,4) mg/ml, insbesondere in einer Konzentration von (3,5 ± 0,3) mg/ml, vorzugsweise in einer Konzentration von (3,5 ± 0,2) mg/ml, bevorzugt in einer Konzentration von (3,5 ± 0,1) mg/ml, besonders bevorzugt in einer Konzentration von etwa 3,5 mg/ml, enthalten. Insbesondere kann die oxybutyninhaltige Zusammensetzung das Natriumchlorid, berechnet als Natrium, in einer Konzentration von 3,5 mg/ml enthalten.

Was den erfindungsgemäß eingestellten bzw. vorliegenden pH-Wert der oxybutyninhaltige Zusammensetzung anbelangt, so kann dieser unter Verwendung von bzw. mittels Salzsäure eingestellt werden.

Erfindungsgemäß ist es dabei bevorzugt, dass die oxybutyninhaltige Zusammensetzung auf einen pH-Wert im Bereich von 3,8 bis 4,5 eingestellt wird bzw. einen diesbezüglichen pH-Wert im Bereich von 3,8 bis 4,5 aufweist, wie zuvor angeführt. Hierdurch wird die Stabilität der oxybutyninhaltige Zusammensetzung weiterführend verbessert.

Was die im Rahmen des erfindungsgemäßen Verfahrens eingesetzte Kolbenspritze anbelangt, so ist es erfindungsgemäß insbesondere vorgesehen, dass diese ein Volumen, insbesondere ein Aufnahmevolumen für die oxybutyninhaltige Zusammensetzung, im Bereich von 4 ml bis 25 ml, insbesondere im Bereich von 6 ml bis 20 ml, vorzugsweise im Bereich von 8 ml bis 15 ml, bevorzugt im Bereich von 9 ml bis 12 ml, besonders bevorzugt von etwa 10 ml, aufweist. Insbesondere kann die Kolbenspritze ein Volumen von 10 ml aufweisen. Das diesbezügliche Volumen bezieht sich dabei insbesondere auf das entsprechende Aufnahmevolumen des Spritzenkörpers bzw. Spritzenzylinders für die oxybutyninhaltige Zusammensetzung.

Erfindungsgemäß kann die Kolbenspritze die oxybutyninhaltige Zusammensetzung in einer Menge (Volumen) im Bereich von 4 ml bis 25 ml, insbesondere im Bereich von 6 ml bis 20 ml, vorzugsweise im Bereich von 8 ml bis 15 ml, bevorzugt im Bereich von 9 ml bis 12 ml, besonders bevorzugt von etwa 10 ml, aufweisen bzw. enthalten. Insbesondere kann die Kolbenspritze die oxybutyninhaltige Zusammensetzung in einer Menge von 10 ml aufweisen bzw. enthalten.

Erfindungsgemäß wird insbesondere eine Kolbenspritze mit einem Volumen, insbesondere mit einem Aufnahmevolumen für die oxybutyninhaltige Zusammensetzung, von 10 ml eingesetzt, wobei die Kolbenspritze die oxybutyninhaltige Zusammensetzung in einer Menge (Volumen) von 10 ml aufweist bzw. enthält.

Gemäß der vorliegenden Erfindung ist der Kolbenstopfen insbesondere aus einem Chlorbutylkautschuk oder aus einem Brombutylkautschuk, vorzugsweise aus einem Brombutylkautschuk, gebildet.

Erfindungsgemäß kann es zudem vorgesehen sein, dass die Kolbenspritze, insbesondere der Spritzenzylinder, eine erste Öffnung, insbesondere zur Aufnahme eines Spritzenkolbens, aufweist. Im Allgemeinen ist der erfindungsgemäß vorgesehene Kolbenstopfen dabei an dem zum Innenraum des Spritzenkörpers, insbesondere Spritzenzylinders, positionierten Ende des Spritzenkolbens angebracht bzw. fixiert.

Erfindungsgemäß kann es gemäß einer bevorzugten Ausführungsform vorgesehen sein, dass die Kolbenspritze einen Spritzenkolben aufweist, wobei der Spritzenkolben aus Polypropylen oder aus einem Cycloolefin-(Co-)Polymer (COP bzw. COC) gebildet sein kann. Insbesondere kann der Spritzenkolben aus einem Cycloolefin-(Co-)Polymer (COP bzw. COC) gebildet sein. Hierdurch werden die Eigenschaften der Kolbenspritze im Rahmen der Dampfsterilisation weiterführend verbessert. Zudem ist es anwendungs- und produktionstechnisch von Vorteil, wenn der Spritzenkörper einerseits und der Spritzenkolben andererseits der erfindungsgemäß eingesetzten Kolbenspritze aus demselben Material in Form von Cycloolefin-(Co-)Polymer (COP bzw. COC) gebildet sind.

Weiterhin ist es erfindungsgemäß insbesondere vorgesehen, dass die Kolbenspritze eine zweite Öffnung, insbesondere Austragsöffnung, vorzugsweise zum Austragen bzw. Applizieren der oxybutyninhaltigen Zusammensetzung, aufweist.

Erfindungsgemäß kann die Kolbenspritze zudem ein Verschlusselement, insbesondere eine Verschlusskappe, vorzugsweise zum Verschließen der zweiten Öffnung, aufweisen. Dabei ist es erfindungsgemäß insbesondere vorgesehen, dass das Verschlusselement aus einem Halogenbutylkautschuk, insbesondere aus einem Chlorbutylkautschuk oder aus einem Brombutylkautschuk, vorzugsweise aus einem Brombutylkautschuk, gebildet ist.

Erfindungsgemäß ist es somit vorzugsweise vorgesehen, dass die Kolbenspritze ein Verschlusselement, insbesondere eine Verschlusskappe, aus einem Brombutylkautschuk aufweist. Hierzu kann ein gleichermaßen der zuvor für den Kolbenstopfen angeführter bzw. eingesetzter Brombutylkautschuk verwendet werden. Durch die Ausbildung auch des Verschlusselements in Form eines Brombutylkautschuks wird die Kompatibilität der insgesamt eingesetzten Komponenten bzw. Stabilität der erfindungsgemäß sterilisierten oxybutyninhaltigen Zusammensetzung bei der Sterilisation weiterführend verbessert, insbesondere was die Verminderung der Bildung von Abbauprodukten des Oxybutyninhydrochlorids anbelangt. Diesbezüglich kann auch auf die Ausführungen zu dem erfindungsgemäß eingesetzten Kolbenstopfen verwiesen werden. Für das Verschlusselement können gleichermaßen markt- bzw. handelsübliche Materialien eingesetzt werden (wie beispielsweise Dätwyler FM252-2 Brombutyl Grad Eu.P. 3.2.9).

Erfindungsgemäß ist es insbesondere vorgesehen, dass die Kolbenspritze mit der darin eingebrachten oxybutyninhaltigen Zusammensetzung bei der Dampfsterilisation mit dem Verschlusselement an der zweiten Öffnung der Kolbenspritze verschlossen ist.

Diesbezüglich ist es erfindungsgemäß insgesamt vorgesehen, dass im Rahmen der Dampfsterilisation vorzugsweise die vollständige Einheit als solche, welche die mit der oxybutyninhaltigen Zusammensetzung befüllte Kolbenspritze mit dem diesbezüglichen Spritzenkörper, den Kolben- bzw. Spritzenstopfen, das Verschlusselement sowie gegebenenfalls den Spritzenkolben umfasst, sterilisiert wird.

Erfindungsgemäß wird die Dampfsterilisation im Allgemeinen unter Temperaturbeaufschlagung (Erwärmung) durchgeführt. Diesbezüglich hat es sich erfindungsgemäß als vorteilhaft erwiesen, wenn die Dampfsterilisation bei Temperaturen von mindestens 105 °C, insbesondere mindestens 110 °C, vorzugsweise mindestens 115 °C, bevorzugt mindestens 120 °C, durchgeführt wird. Insbesondere sollte Dampfsterilisation bei Temperaturen von höchstens 160 °C, insbesondere höchstens 150 °C, vorzugsweise höchstens 140° C bevorzugt höchstens 130° C, durchgeführt werden.

Im Allgemeinen kann es erfindungsgemäß vorgesehen sein, dass die Dampfsterilisation bei Temperaturen im Bereich von 105 °C bis 160 °C, insbesondere im Bereich von 110 °C bis 150 °C, vorzugsweise im Bereich von 115 °C bis 140 °C, bevorzugt im Bereich von 120 °C bis 130° C, besonders bevorzugt bei etwa 121 °C, durchgeführt wird. Insbesondere kann die Dampfsterilisation im Rahmen des erfindungsgemäßen Verfahrens bei 121 °C durchgeführt werden.

Bei den zuvor genannten Temperaturen kann eine wirkungsvolle Sterilisation durchgeführt werden, wobei zudem eine entsprechende Materialschonung gewährleistet ist. Die in Rede stehenden Temperaturen beziehen sich dabei insbesondere auf die kälteste Stelle bzw. Position in einem Sterilisationsraum einer diesbezüglich eingesetzten Sterilisationsvorrichtung.

Erfindungsgemäß kann es vorgesehen sein, dass die Dampfsterilisation für einen Zeitraum von mindestens 1 min, insbesondere von mindestens 2 min, vorzugsweise von mindestens 3 min, bevorzugt von mindestens 5 min, besonders bevorzugt von mindestens 8 min, ganz besonders bevorzugt von mindestens 10min, weiter bevorzugt von mindestens 15 min, noch weiter bevorzugt von mindestens 17 min, durchgeführt wird.

Zudem kann es erfindungsgemäß vorgesehen sein, dass die Dampfsterilisation für einen Zeitraum von höchstens 500 min, insbesondere von höchstens 400 min, vorzugsweise von höchstens 200 min, bevorzugt von höchstens 100 min, besonders bevorzugt von höchstens 60 min, ganz besonders bevorzugt von höchstens 45 min, weiter bevorzugt von höchstens 30 min, noch weiter bevorzugt von höchstens 25 min, durchgeführt wird.

Erfindungsgemäß kann die Dampfsterilisation im Allgemeinen für einen Zeitraum im Bereich von 1 min bis 500 min, insbesondere im Bereich von 2 min bis 400 min, vorzugsweise im Bereich von 3 min bis 200 min, bevorzugt im Bereich von 5 min bis 100 min, besonders bevorzugt im Bereich von 8 min bis 60 min, ganz besonders bevorzugt im Bereich von 10 min bis 45 min, weiter bevorzugt im Bereich von 15 min bis 30 min, noch weiter bevorzugt im Bereich von 17 min bis 25 min, besonders weiter bevorzugt für etwa 20 min, durchgeführt werden. Erfindungsgemäß kann somit eine effektive Sterilisation bereits nach relativ kurzen Sterilisationsdauern erreicht werden.

Erfindungsgemäß kann es zudem vorgesehen sein, dass die Dampfsterilisation unter Druckbeaufschlagung bzw. unter erhöhtem Druck durchgeführt wird. Hierdurch kann die Sterilisationseffizienz weiterführend erhöht werden.

Unter dem Begriff "Druckbeaufschlagung" bzw. "erhöhter Druck" ist im Rahmen der vorliegenden Erfindung insbesondere der Druck der bei der Dampfsterilisation vorliegenden Gasphase zu verstehen, welcher höher ist als der Druck unter Standardbedingungen (1,013 bar bei 25 °C) oder als der Druck der umgebenden Atmosphäre (im Allgemeinen ungefähr 1 bar). Ein erhöhter Druck kann folglich durch einen positiven Betrag des Relativdrucks charakterisiert bzw. erfasst werden. Unter dem "Relativdruck" eines Systems wird im Allgemeinen insbesondere die Druckdifferenz zwischen dem Absolutdruck der Gasphase des betreffenden Systems (beispielsweise dem Druck innerhalb eines Autoklaven) und dem Absolutdruck der umgebenden Atmosphäre verstanden. Was den erfindungsgemäß verwendeten Begriff "Absolutdruck" anbelangt, so wird hierunter insbesondere der Druck gegenüber dem Druck Null im leeren Raum (Vakuum) verstanden.

Erfindungsgemäß kann die Dampfsterilisation insbesondere bei einem Relativdruck im Bereich von 0,1 bar bis 10 bar, insbesondere im Bereich von 0,5 bar bis 5 bar, vorzugsweise im Bereich von 1 bar bis 3,5 bar, bevorzugt im Bereich von 1,5 bar bis 3 bar, durchgeführt werden. Insbesondere kann die Dampfsterilisation bei einem Absolutdruck im Bereich von 1,1 bar bis 10 bar, insbesondere im Bereich von 1,5 bar bis 6 bar, vorzugsweise im Bereich von 2 bar bis 4,5 bar, durchgeführt werden. Bei dem in Rede stehenden Druckbereich wird auch eine ausreichende Kondensation des Wasserdampfs auf dem zu sterilisierenden Material gewährleistet, einhergehend mit einem entsprechend guten Energie- bzw. Wärmeübertrag.

Im Allgemeinen kann es im Rahmen der vorliegenden Erfindung vorgesehen sein, dass die Dampfsterilisation in Gegenwart einer insbesondere reinen Wasserdampf enthaltenden Atmosphäre bzw. als Sattdampfverfahren durchgeführt wird. Demgegenüber kann es erfindungsgemäß aber auch vorgesehen sein, dass die Dampfsterilisation in Gegenwart eines Wasserdampf/Gas-Gemisches, insbesondere eines Gemisches von Wasserdampf mit einem Inertgas, wie Stickstoff, und/oder Sauerstoff, vorzugsweise eines Wasserdampf/Luft-Gemisches, durchgeführt wird.

Unter Sattdampf ist dabei insbesondere eine gesättigte Atmosphäre aus reinem Wasserdampf zu verstehen (wobei reiner Wasserdampf, insbesondere gemäß der europäischen Norm EN 285, bis zu 3,5 Vol.-% nicht kondensierbare Gase enthalten kann, wie Sauerstoff, Stickstoff etc.), während eine reinen Wasserdampf enthaltende Atmosphäre beispielsweise auch eine Atmosphäre aus überhitztem Dampf sein kann.

Erfindungsgemäß sollte die Dampfsterilisation in einer vorzugsweise geschlossenen Sterilisationsvorrichtung, insbesondere in einem gasdicht verschlossenen Druckbehälter, vorzugsweise in einer Autoklaviervorrichtung (Autoklav), durchgeführt werden. Die entsprechenden Apparaturen bzw. Vorrichtungen sind dem Fachmann wohlbekannt, so dass es hierzu keinerlei weiteren Ausführungen bedarf.

Im Rahmen der vorliegenden Erfindung kann es insbesondere vorgesehen sein, dass bei der Dampfsterilisation der Wasserdampf aus destilliertem bzw. voll entsalztem Wasser erzeugt wird. Insbesondere kann bei der Dampfsterilisation die Druckluft aus steriler Luft erzeugt werden, insbesondere wobei die Luft mittels Durchströmen von Filtern gereinigt bzw. sterilisiert wird. Diesbezüglich können insbesondere Partikelfilter, wie beispielsweise HEPA-Filter oder dergleichen, eingesetzt werden. Hierdurch wird insbesondere verhindert, dass keine zusätzlichen Mikroorganismen in die Sterilisationseinrichtung eingetragen werden.

Gemäß einer erfindungsgemäß bevorzugten Ausführungsform kann die Dampfsterilisation unter *overkill*-Bedingungen und/oder bei etwa 121 °C und/oder für einen Zeitraum von mindestens 15 min, insbesondere von mindestens 20 min, vorzugsweise von etwa 20 min, durchgeführt werden. Insbesondere kann die Dampfsterilisation diesbezüglich bei 121 °C und für einen Zeitraum von 20 min durchgeführt werden. Hierdurch werden besonders gute Sterilisationsergebnisse erhalten. Die erfindungsgemäß angeführten *overkill*-Bedingungen stellen dabei insbesondere auf eine Weiterentwicklung des in Pharmacopoea Europaea (Ph. Eur.), Kapitel 5.1.1 (07/2017:50101) angeführten Referenzverfahrens zur Dampfsterilisation ab.

Erfindungsgemäß hat es sich zudem als vorteilhaft erwiesen, wenn die Dampfsterilisation gemäß Pharmacopoea Europaea (Ph. Eur.), Kapitel 5.1.1 (07/2017:50101) durchgeführt wird. Diesbezüglich kommen erfindungsgemäß gleichermaßen insbesondere *overkill*-Bedingungen zum Einsatz. Insbesondere kann diesbezüglich bei einer Temperatur von etwa 121 °C bzw. für einen Zeitraum von mindestens 15 min, insbesondere von mindestens 20 min, vorzugsweise von etwa 20 min, verfahren bzw. dampfsterilisiert werden. Hierzu kann auch auf die entsprechenden Ausführungen in Pharmacopoea Europaea (Ph. Eur.), Kapitel 5.1.1 (07/2017:50101) verwiesen werden, wobei der diesbezügliche Offenbarungsgehalt hiermit durch Bezugnahme vollumfänglich eingeschlossen ist.

Erfindungsgemäß kann zudem auch derart vorgegangen werden, dass die Dampfsterilisation mehrzyklisch durchgeführt wird. Diesbezüglich kann zumindest der abschließende bzw. terminale Zyklus unter mindestens einer bzw. einem der zuvor definierten (Dampfsterilisations-)Bedingungen bzw. Parameter durchgeführt werden. In diesem Zusammenhang kann zumindest der abschließende bzw. terminale Zyklus bzw. die terminale Dampfsterilisation unter *overkill*-Bedingungen und/oder bei etwa 121 °C und/oder für einen Zeitraum von mindestens 15 min, insbesondere von mindestens 20 min, vorzugsweise von etwa 20 min, durchgeführt werden. Insbesondere wird auch diesbezüglich bei 121 °C und für einen Zeitraum von 20 min verfahren bzw. dampfsterilisiert.

Gemäß einer erfindungsgemäßen Ausführungsform kann es grundsätzlich vorgesehen sein, dass die mit der oxybutyninhaltigen Zusammensetzung befüllte Kolbenspritze, insbesondere vor Durchführung der Dampfsterilisation, in eine insbesondere wasser- und/oder keimdicht verschlossene und/oder versiegelte Verpackung, insbesondere Bereitschaftspackung, eingebracht worden ist bzw. eingebracht vorliegt. In diesem Zusammenhang ist es erfindungsgemäß insbesondere vorgesehen, dass zumindest ein Teil der Verpackung wenigstens wasserdampfdurchlässig, insbesondere wasserdampf- und/oder gasdurchlässig, ausgebildet ist. Hierdurch wird weiterhin eine gute Sterilisation auch der eingebrachten bzw. verpackten Kolbenspritze mit der oxybutyninhaltigen Zusammensetzung gewährleistet. Erfindungsgemäß verhält es sich dabei insbesondere derart, dass die gegebenenfalls vorhandene Verpackung bei Durchführung der Dampfsterilisation gleichermaßen (mit-)sterilisiert wird.

Gemäß einer erfindungsgemäßen Ausführungsform kann es zudem vorgesehen sein, dass eine Vielzahl von mit der oxybutyninhaltigen Zusammensetzung befüllte Kolbenspritzen gleichzeitig bzw. gemeinsam sterilisiert werden. Hierdurch können hohe Sterilisationsdurchsätze erreicht werden. In diesem Zusammenhang kann es insbesondere vorgesehen sein, dass vorzugsweise während der Durchführung der Dampfsterilisation mehrere Kolbenspritzen auf einem (Spritzen-)Träger zusammengeführt bzw. zusammengefasst sind.

Im Rahmen des erfindungsgemäßen Verfahrens werden insgesamt hervorragende Sterilisationsergebnisse erhalten. In diesem Zusammenhang ist es erfindungsgemäß insbesondere vorgesehen, dass die nach Dampfsterilisation erhaltene sterile oxybutyninhaltige Zusammensetzung bzw. die mit der oxybutyninhaltigen Zusammensetzung befüllte nach Dampfsterilisation erhaltene sterile Kolbenspritze einen SAL-Wert (*Sterility Assurance Level*) von mindestens 10⁻⁵, insbesondere mindestens 10⁻⁶, vorzugsweise mindestens 10⁻⁷, aufweist. Insbesondere kann es erfindungsgemäß vorgesehen sein, dass die dampfsterilisierte bzw. sterile oxybutyninhaltige Zusammensetzung bzw. die nach Dampfsterilisation erhaltene und mit der oxybutyninhaltigen Zusammensetzung befüllte Kolbenspritze steril gemäß der DIN EN 566-1 ist.

Für weitere Ausführungen zu der dem erfindungsgemäßen Verfahren zugrundeliegenden Dampfsterilisation kann insbesondere auf die DE 10 2017 104 931 A1 sowie auf die zu derselben Patentfamilie gehörende WO 2018/145799 A1 verwiesen werden, wobei der diesbezügliche Offenbarungsgehalt hiermit durch Bezugnahme vollumfänglich eingeschlossen ist. Zudem kann auch auf die DE 10 2011 105 840 A1 sowie auf die zu derselben Patentfamilie gehörende WO 2012/136313 A2 bzw. auf die US 2014/093422 A1 verwiesen werden, deren jeweiliger Offenbarungsgehalt hiermit gleichermaßen durch Bezugnahme vollumfänglich eingeschlossen ist.

Wie zuvor angeführt, wird im Rahmen des erfindungsgemäßen Verfahrens eine sterile oxybutyninhaltige Zusammensetzung mit hoher Stabilität bereitgestellt. Insbesondere weist die sterile oxybutyninhaltige Zusammensetzung einen geringen Gehalt an Verunreinigungen bzw. Abbauprodukten auf.

In diesem Zusammenhang verhält es sich im Rahmen der vorliegenden Erfindung insbesondere derart, dass die nach der Dampfsterilisation erhaltene sterile oxybutyninhaltige Zusammensetzung einen Gehalt an Abbauprodukt(en) des Oxybutynins, insbesondere einen Gehalt an Phenylcyclohexylglykolsäure, von höchstens 10 Gew.-%, insbesondere höchstens 7,5 Gew.-%, vorzugsweise höchstens 5 Gew.-%, bevorzugt höchstens 2 Gew.-%, besonders bevorzugt höchstens 1 Gew.-%, bezogen auf Oxybutyninhydrochlorid, aufweist.

Gemäß dem vorliegenden Aspekt betrifft die vorliegende Erfindung auch ein Verfahren zur Herstellung einer sterilen oxybutyninhaltigen Zusammensetzung in einer vorzugsweise anwendungsfertigen Kolbenspritze, insbesondere Einweg-Kolbenspritze, und/oder zur Herstellung einer vorzugsweise anwendungsfertigen, sterilen, mit einer oxybutyninhaltigen Zusammensetzung befüllten Kolbenspritze, insbesondere Einweg-Kolbenspritze, insbesondere ein wie zuvor definiertes Verfahren,
wobei die Kolbenspritze einen Spritzenkörper, insbesondere Spritzenzylinder, einerseits und einen Spritzenkolben mit einem Kolbenstopfen andererseits aufweist, wobei der Spritzenkörper aus einem Cycloolefin-(Co-)Polymer (COP bzw. COC) gebildet ist und wobei der Kolbenstopfen aus einem Halogenbutylkautschuk, insbesondere aus einem Chlorbutylkautschuk oder aus einem Brombutylkautschuk, vorzugsweise aus einem Brombutylkautschuk, gebildet ist;
wobei das Verfahren die nachfolgenden Schritte umfasst:
a) Befüllen der Kolbenspritze mit der oxybutyninhaltigen Zusammensetzung und/oder Bereitstellen der oxybutyninhaltigen Zusammensetzung in der Kolbenspritze,
   wobei das Oxybutynin in Form des Hydrochlorids eingesetzt wird, die oxybutyninhaltige Zusammensetzung als wässrige Zusammensetzung ausgebildet wird und die oxybutyninhaltige Zusammensetzung in Kombination und jeweils in wirksamen, insbesondere pharmazeutisch wirksamen Mengen,
   - Oxybutyninhydrochlorid (Oxybutynin-HCI) in einer Konzentration von (1 ± 0,4) mg/ml, insbesondere in einer Konzentration von (1 ± 0,3) mg/ml, vorzugsweise in einer Konzentration von (1 ± 0,2) mg/ml, bevorzugt in einer Konzentration von (1 ± 0,1) mg/ml, besonders bevorzugt in einer Konzentration von etwa 1 mg/ml, und
   - Natriumchlorid, berechnet als Natrium, in einer Konzentration von (3,5 ± 0,4) mg/ml, insbesondere in einer Konzentration von (3,5 ± 0,3) mg/ml, vorzugsweise in einer Konzentration von (3,5 ± 0,2) mg/ml, bevorzugt in einer Konzentration von (3,5 ± 0,1) mg/ml, besonders bevorzugt in einer Konzentration von etwa 3,5 mg/ml
   enthält,
   wobei die oxybutyninhaltige Zusammensetzung auf einen pH-Wert im Bereich von 3 bis 5, insbesondere im Bereich von 3,8 bis 4,5, eingestellt wird und/oder wobei die oxybutyninhaltige Zusammensetzung einen pH-Wert im Bereich von 3 bis 5, insbesondere im Bereich von 3,8 bis 4,5, aufweist;
   und nachfolgend
b) Dampfsterilisation, insbesondere Wasserdampfsterilisation, der oxybutyninhaltigen Zusammensetzung in der Kolbenspritze und/oder der mit der oxybutyninhaltigen Zusammensetzung befüllten Kolbenspritze.

Weiterhin betrifft die vorliegende Erfindung gemäß diesem Aspekt der vorliegenden Erfindung auch ein Verfahren zur Herstellung einer sterilen oxybutyninhaltigen Zusammensetzung in einer vorzugsweise anwendungsfertigen Kolbenspritze, insbesondere Einweg-Kolbenspritze, und/oder zur Herstellung einer vorzugsweise anwendungsfertigen, sterilen, mit einer oxybutyninhaltigen Zusammensetzung befüllten Kolbenspritze, insbesondere Einweg-Kolbenspritze, insbesondere ein wie zuvor definiertes Verfahren,
wobei die Kolbenspritze einen Spritzenkörper, insbesondere Spritzenzylinder, einerseits und einen Spritzenkolben mit einem Kolbenstopfen andererseits aufweist, wobei der Spritzenkörper aus einem Cycloolefin-(Co-)Polymer (COP bzw. COC) gebildet ist und wobei der Kolbenstopfen aus einem Halogenbutylkautschuk, insbesondere aus einem Chlorbutylkautschuk oder aus einem Brombutylkautschuk, vorzugsweise aus einem Brombutylkautschuk, gebildet ist;
wobei das Verfahren die nachfolgenden Schritte umfasst:
a) Befüllen der Kolbenspritze mit der oxybutyninhaltigen Zusammensetzung und/oder Bereitstellen der oxybutyninhaltigen Zusammensetzung in der Kolbenspritze,
   wobei das Oxybutynin in Form des Hydrochlorids eingesetzt wird, die oxybutyninhaltige Zusammensetzung als wässrige Zusammensetzung ausgebildet wird und die oxybutyninhaltige Zusammensetzung in Kombination und jeweils in wirksamen, insbesondere pharmazeutisch wirksamen Mengen,
   - Oxybutyninhydrochlorid (Oxybutynin-HCI) in einer Konzentration von etwa 1 mg/ml, und
   - Natriumchlorid, berechnet als Natrium, in einer Konzentration von etwa 3,5 mg/ml
   enthält,
   wobei die oxybutyninhaltige Zusammensetzung auf einen pH-Wert im Bereich von 3,8 bis 4,5 eingestellt wird und/oder wobei die oxybutyninhaltige Zusammensetzung einen pH-Wert im Bereich von 3,8 bis 4,5 aufweist; und nachfolgend
b) Dampfsterilisation, insbesondere Wasserdampfsterilisation, der oxybutyninhaltigen Zusammensetzung in der Kolbenspritze und/oder der mit der oxybutyninhaltigen Zusammensetzung befüllten Kolbenspritze.

Weiterhin betrifft die vorliegende Erfindung gemäß diesem Aspekt auch ein Verfahren zur Herstellung einer sterilen oxybutyninhaltigen Zusammensetzung in einer vorzugsweise anwendungsfertigen Kolbenspritze, insbesondere Einweg-Kolbenspritze, und/oder zur Herstellung einer vorzugsweise anwendungsfertigen, sterilen, mit einer oxybutyninhaltigen Zusammensetzung befüllten Kolbenspritze, insbesondere Einweg-Kolbenspritze, insbesondere ein wie zuvor definiertes Verfahren,
wobei die Kolbenspritze einen Spritzenkörper, insbesondere Spritzenzylinder, einerseits und einen Spritzenkolben mit einem Kolbenstopfen andererseits aufweist, wobei der Spritzenkörper aus einem Cycloolefin-(Co-)Polymer (COP bzw. COC) gebildet ist und wobei der Kolbenstopfen aus einem Halogenbutylkautschuk, insbesondere aus einem Chlorbutylkautschuk oder aus einem Brombutylkautschuk, vorzugsweise aus einem Brombutylkautschuk, gebildet ist;
wobei das Verfahren die nachfolgenden Schritte umfasst:
a) Befüllen der Kolbenspritze mit der oxybutyninhaltigen Zusammensetzung und/oder Bereitstellen der oxybutyninhaltigen Zusammensetzung in der Kolbenspritze,
   wobei das Oxybutynin in Form des Hydrochlorids eingesetzt wird, die oxybutyninhaltige Zusammensetzung als wässrige Zusammensetzung ausgebildet wird und die oxybutyninhaltige Zusammensetzung in Kombination und jeweils in wirksamen, insbesondere pharmazeutisch wirksamen Mengen,
   - Oxybutyninhydrochlorid (Oxybutynin-HCI) in einer Konzentration von etwa 1 mg/ml, und
   - Natriumchlorid, berechnet als Natrium, in einer Konzentration von etwa 3,5 mg/ml
   enthält,
   wobei die oxybutyninhaltige Zusammensetzung auf einen pH-Wert im Bereich von 3,8 bis 4,5 eingestellt wird und/oder wobei die oxybutyninhaltige Zusammensetzung einen pH-Wert im Bereich von 3,8 bis 4,5 aufweist;
   und nachfolgend
b) Dampfsterilisation, insbesondere Wasserdampfsterilisation, der oxybutyninhaltigen Zusammensetzung in der Kolbenspritze und/oder der mit der oxybutyninhaltigen Zusammensetzung befüllten Kolbenspritze,
   wobei die Dampfsterilisation unter *overkill*-Bedingungen und/oder bei etwa 121 °C und/oder für einen Zeitraum von mindestens 15 min, insbesondere von mindestens 20 min, vorzugsweise von etwa 20 min, durchgeführt wird und/oder
   wobei die Dampfsterilisation gemäß Pharmacopoea Europaea (Ph. Eur.), Kapitel 5.1.1 (07/2017:50101) durchgeführt wird, insbesondere unter *overkill-*Bedingungen und/oder bei etwa 121 °C und/oder für einen Zeitraum von mindestens 15 min, insbesondere von mindestens 20 min, vorzugsweise von etwa 20 min.

In Bezug auf die Ausgestaltung des erfindungsgemäßen Verfahrens gemäß dem vorliegenden Aspekt kann auch auf die nachfolgenden Ausführungen zu den weiteren erfindungsgemäßen Aspekten verwiesen werden, welche entsprechend gelten.

Darüber hinaus ist Gegenstand der vorliegenden Erfindung - gemäß einem **weiteren** Aspekt der vorliegenden Erfindung - auch die sterile oxybutyninhaltige Zusammensetzung, insbesondere zur Verwendung bei der prophylaktischen und/oder therapeutischen Behandlung von neurogenen Blasenfunktionsstörungen, insbesondere neurogenen Blasenentleerungsstörungen, vorzugsweise von mit einer Detrusorhyperaktivität und/oder einer Detrusor-Sphinkter-Dyssynergie einhergehenden neurogenen Blasenfunktionsstörungen,
wobei die oxybutyninhaltige Zusammensetzung nach dem erfindungsgemäßen Verfahren erhältlich ist; und/oder
wobei die oxybutyninhaltige Zusammensetzung nach dem erfindungsgemäßen Verfahren dampfsterilisiert, insbesondere wasserdampfsterilisiert, worden ist.

Gemäß diesem Aspekt betrifft die vorliegende Erfindung auch die sterile oxybutyninhaltige Zusammensetzung, insbesondere zur Verwendung bei der prophylaktischen und/oder therapeutischen Behandlung von neurogenen Blasenfunktionsstörungen, insbesondere neurogenen Blasenentleerungsstörungen, vorzugsweise von mit einer Detrusorhyperaktivität und/oder einer Detrusor-Sphinkter-Dyssynergie einhergehenden neurogenen Blasenfunktionsstörungen, insbesondere die zuvor definierte Zusammensetzung,
wobei die oxybutyninhaltige Zusammensetzung in einer vorzugsweise anwendungsfertigen Kolbenspritze, insbesondere Einweg-Kolbenspritze, vorliegt,
wobei die Kolbenspritze einen Spritzenkörper, insbesondere Spritzenzylinder, einerseits und einen Spritzenkolben mit einem Kolbenstopfen andererseits aufweist, wobei der Spritzenkörper aus einem Cycloolefin-(Co-)Polymer (COP bzw. COC) gebildet ist und wobei der Kolbenstopfen aus einem Halogenbutylkautschuk gebildet ist;
wobei die oxybutyninhaltige Zusammensetzung das Oxybutynin in Form des Hydrochlorids enthält, die oxybutyninhaltige Zusammensetzung als wässrige Zusammensetzung ausgebildet ist und die oxybutyninhaltige Zusammensetzung in Kombination und jeweils in wirksamen, insbesondere pharmazeutisch wirksamen Mengen,
- Oxybutyninhydrochlorid (Oxybutynin-HCI) in einer Konzentration von (1 ± 0,5) mg/ml und
- Natriumchlorid, berechnet als Natrium, in einer Konzentration von (3,5 ± 0,5) mg/ml
enthält,
wobei die oxybutyninhaltige Zusammensetzung auf einen pH-Wert im Bereich von 3 bis 5, insbesondere im Bereich von 3,8 bis 4,5, eingestellt ist und/oder wobei die oxybutyninhaltige Zusammensetzung einen pH-Wert im Bereich von 3 bis 5, insbesondere im Bereich von 3,8 bis 4,5, aufweist; und
wobei die in der Kolbenspritze enthaltene oxybutyninhaltige Zusammensetzung einschließlich der Kolbenspritze dampfsterilisiert, insbesondere wasserdampfsterilisiert, ist.

In diesem Zusammenhang kann die oxybutyninhaltige Zusammensetzung das Oxybutyninhydrochlorid (Oxybutynin-HCI) in einer Konzentration von (1 ± 0,4) mg/ml, insbesondere in einer Konzentration von (1 ± 0,3) mg/ml, vorzugsweise in einer Konzentration von (1 ± 0,2) mg/ml, bevorzugt in einer Konzentration von (1 ± 0,1) mg/ml, besonders bevorzugt in einer Konzentration von etwa 1 mg/ml, enthalten.

Weiterhin kann die oxybutyninhaltige Zusammensetzung das Natriumchlorid, berechnet als Natrium, in einer Konzentration von (3,5 ± 0,4) mg/ml, insbesondere in einer Konzentration von (3,5 ± 0,3) mg/ml, vorzugsweise in einer Konzentration von (3,5 ± 0,2) mg/ml, bevorzugt in einer Konzentration von (3,5 ± 0,1) mg/ml, besonders bevorzugt in einer Konzentration von etwa 3,5 mg/ml, enthalten.

Erfindungsgemäß ist es insbesondere bevorzugt, dass der Kolbenstopfen aus einem Chlorbutylkautschuk oder aus einem Brombutylkautschuk, vorzugsweise aus einem Brombutylkautschuk, gebildet ist.

Im Allgemeinen kann die oxybutyninhaltige Zusammensetzung nach der Erfindung unter mindestens einer bzw. einem der zuvor für das erfindungsgemäße Verfahren definierten (Dampfsterilisations-)Bedingungen bzw. Parameter dampfsterilisiert sein.

Erfindungsgemäß verhält es sich zudem insbesondere derart, dass die oxybutyninhaltige Zusammensetzung unter *overkill*-Bedingungen und/oder bei etwa 121 °C und/oder für einen Zeitraum von mindestens 15 min, insbesondere von mindestens 20 min, vorzugsweise von etwa 20 min, dampfsterilisiert ist.

Insbesondere kann es erfindungsgemäß vorgesehen sein, dass die oxybutyninhaltige Zusammensetzung gemäß Pharmacopoea Europaea (Ph. Eur.), Kapitel 5.1.1 (07/2017:50101) dampfsterilisiert ist, insbesondere unter *overkill-*Bedingungen und/oder bei etwa 121 °C und/oder für einen Zeitraum von mindestens 15 min, insbesondere von mindestens 20 min, vorzugsweise von etwa 20 min.

Insbesondere kann die nach Dampfsterilisation erhaltene sterile oxybutyninhaltige Zusammensetzung bzw. die mit der oxybutyninhaltigen Zusammensetzung befüllte nach Dampfsterilisation erhaltene sterile Kolbenspritze einen SAL-Wert (*Sterility* Assurance) von mindestens 10⁻⁵, insbesondere mindestens 10⁻⁶, vorzugsweise mindestens 10⁻⁷, aufweisen.

Wie zuvor angeführt, weist die sterile oxybutyninhaltige Zusammensetzung eine hohe Stabilität auf. Insbesondere kann die nach Dampfsterilisation erhaltene sterile oxybutyninhaltige Zusammensetzung einen Gehalt an Abbauprodukt(en) des Oxybutynins, insbesondere einen Gehalt an Phenylcyclohexylglykolsäure, von höchstens 10 Gew.-%, insbesondere höchstens 7,5 Gew.-%, vorzugsweise höchstens 5 Gew.-%, bevorzugt höchstens 2 Gew.-%, besonders bevorzugt höchstens 1 Gew.-%, bezogen auf Oxybutyninhydrochlorid, aufweisen.

Wie zuvor angeführt, kann die erfindungsgemäße Zusammensetzung insbesondere zur prophylaktischen bzw. therapeutischen Behandlung von neurogenen Blasenfunktionsstörungen eingesetzt werden. Somit betrifft die vorliegende Erfindung auch die Verwendung der sterilen oxybutyninhaltigen Zusammensetzung (zur Herstellung eines Medikaments) zur Behandlung von neurogenen Blasenfunktionsstörungen. Insbesondere betrifft die vorliegende Erfindung auch die erfindungsgemäße sterile oxybutyninhaltige Zusammensetzung zur Verwendung bei der Behandlung von neurogenen Blasenfunktionsstörungen.

Erfindungsgemäß verhält es sich im Allgemeinen derart, dass die neurogenen Blasenfunktionsstörungen mit einer Detrusorüberaktivität und/oder Detrusor-Sphinkter-Dyssynergie, insbesondere mit überaktivem Detrusor, einhergehen bzw. hierdurch gekennzeichnet sind.

Insbesondere können die neurogenen Blasenfunktionsstörungen mit einer Rückenmarksverletzung, insbesondere einer Querschnittlähmung, Spina bifida, Diabetes, Multiple Sklerose, Schlaganfall oder Morbus Parkinson im Zusammenhang stehen bzw. hierdurch verursacht sein.

Gemäß dem vorliegenden erfindungsgemäßen Aspekt betrifft die vorliegende Erfindung auch eine sterile oxybutyninhaltige Zusammensetzung, insbesondere zur Verwendung bei der prophylaktischen und/oder therapeutischen Behandlung von neurogenen Blasenfunktionsstörungen, insbesondere neurogenen Blasenentleerungsstörungen, vorzugsweise von mit einer Detrusorhyperaktivität und/oder einer Detrusor-Sphinkter-Dyssynergie einhergehenden neurogenen Blasenfunktionsstörungen, insbesondere eine wie zuvor definierte Zusammensetzung,
wobei die oxybutyninhaltige Zusammensetzung in einer vorzugsweise anwendungsfertigen Kolbenspritze, insbesondere Einweg-Kolbenspritze, vorliegt,
wobei die Kolbenspritze einen Spritzenkörper, insbesondere Spritzenzylinder, einerseits und einen Spritzenkolben mit einem Kolbenstopfen andererseits aufweist, wobei der Spritzenkörper aus einem Cycloolefin-(Co-)Polymer (COP bzw. COC) gebildet ist und wobei der Kolbenstopfen aus einem Halogenbutylkautschuk, insbesondere aus einem Chlorbutylkautschuk oder Brombutylkautschuk, vorzugsweise aus einem Brombutylkautschuk, gebildet ist;
wobei die oxybutyninhaltige Zusammensetzung das Oxybutynin in Form des Hydrochlorids enthält, die oxybutyninhaltige Zusammensetzung als wässrige Zusammensetzung ausgebildet ist und die oxybutyninhaltige Zusammensetzung in Kombination und jeweils in wirksamen, insbesondere pharmazeutisch wirksamen Mengen,
- Oxybutyninhydrochlorid (Oxybutynin-HCI) in einer Konzentration von (1 ± 0,4) mg/ml, insbesondere in einer Konzentration von (1 ± 0,3) mg/ml, vorzugsweise in einer Konzentration von (1 ± 0,2) mg/ml, bevorzugt in einer Konzentration von (1 ± 0,1) mg/ml, besonders bevorzugt in einer Konzentration von etwa 1 mg/ml, und
- Natriumchlorid, berechnet als Natrium, in einer Konzentration von (3,5 ± 0,4) mg/ml, insbesondere in einer Konzentration von (3,5 ± 0,3) mg/ml, vorzugsweise in einer Konzentration von (3,5 ± 0,2) mg/ml, bevorzugt in einer Konzentration von (3,5 ± 0,1) mg/ml, besonders bevorzugt in einer Konzentration von etwa 3,5 mg/ml,
enthält,
wobei die oxybutyninhaltige Zusammensetzung auf einen pH-Wert im Bereich von 3 bis 5, insbesondere im Bereich von 3,8 bis 4,5, eingestellt ist und/oder wobei die oxybutyninhaltige Zusammensetzung einen pH-Wert im Bereich von 3 bis 5, insbesondere im Bereich von 3,8 bis 4,5, aufweist; und
wobei die in der Kolbenspritze enthaltene oxybutyninhaltige Zusammensetzung einschließlich der Kolbenspritze dampfsterilisiert, insbesondere wasserdampfsterilisiert, ist.

Gemäß dem vorliegenden Aspekt betrifft die vorliegende Erfindung auch die sterile oxybutyninhaltige Zusammensetzung, insbesondere zur Verwendung bei der prophylaktischen und/oder therapeutischen Behandlung von neurogenen Blasenfunktionsstörungen, insbesondere neurogenen Blasenentleerungsstörungen, vorzugsweise von mit einer Detrusorhyperaktivität und/oder einer Detrusor-Sphinkter-Dyssynergie einhergehenden neurogenen Blasenfunktionsstörungen, insbesondere eine wie zuvor definierte Zusammensetzung,
wobei die oxybutyninhaltige Zusammensetzung in einer vorzugsweise anwendungsfertigen Kolbenspritze, insbesondere Einweg-Kolbenspritze, vorliegt,
wobei die Kolbenspritze einen Spritzenkörper, insbesondere Spritzenzylinder, einerseits und einen Spritzenkolben mit einem Kolbenstopfen andererseits aufweist, wobei der Spritzenkörper aus einem Cycloolefin-(Co-)Polymer (COP bzw. COC) gebildet ist und wobei der Kolbenstopfen aus einem Halogenbutylkautschuk, insbesondere aus einem Chlorbutylkautschuk oder Brombutylkautschuk, vorzugsweise aus einem Brombutylkautschuk, gebildet ist;
wobei die oxybutyninhaltige Zusammensetzung das Oxybutynin in Form des Hydrochlorids enthält, die oxybutyninhaltige Zusammensetzung als wässrige Zusammensetzung ausgebildet ist und die oxybutyninhaltige Zusammensetzung in Kombination und jeweils in wirksamen, insbesondere pharmazeutisch wirksamen Mengen,
- Oxybutyninhydrochlorid (Oxybutynin-HCI) in einer Konzentration von etwa 1 mg/ml und
- Natriumchlorid, berechnet als Natrium, in einer Konzentration von etwa 3,5 mg/ml
enthält,
wobei die oxybutyninhaltige Zusammensetzung auf einen pH-Wert im Bereich von 3,8 bis 4,5, eingestellt ist und/oder wobei die oxybutyninhaltige Zusammensetzung einen pH-Wert im Bereich von 3,8 bis 4,5 aufweist; und
wobei die in der Kolbenspritze enthaltene oxybutyninhaltige Zusammensetzung einschließlich der Kolbenspritze dampfsterilisiert, insbesondere wasserdampfsterilisiert, ist.

Gemäß dem vorliegenden Aspekt betrifft die vorliegende Erfindung weiterhin auch die sterile oxybutyninhaltige Zusammensetzung, insbesondere zur Verwendung bei der prophylaktischen und/oder therapeutischen Behandlung von neurogenen Blasenfunktionsstörungen, insbesondere neurogenen Blasenentleerungsstörungen, vorzugsweise von mit einer Detrusorhyperaktivität und/oder einer Detrusor-Sphinkter-Dyssynergie einhergehenden neurogenen Blasenfunktionsstörungen, insbesondere eine wie zuvor definierte Zusammensetzung,
wobei die oxybutyninhaltige Zusammensetzung in einer vorzugsweise anwendungsfertigen Kolbenspritze, insbesondere Einweg-Kolbenspritze, vorliegt,
wobei die Kolbenspritze einen Spritzenkörper, insbesondere Spritzenzylinder, einerseits und einen Spritzenkolben mit einem Kolbenstopfen andererseits aufweist, wobei der Spritzenkörper aus einem Cycloolefin-(Co-)Polymer (COP bzw. COC) gebildet ist und wobei der Kolbenstopfen aus einem Halogenbutylkautschuk, insbesondere aus einem Chlorbutylkautschuk oder Brombutylkautschuk, vorzugsweise aus einem Brombutylkautschuk, gebildet ist;
wobei die oxybutyninhaltige Zusammensetzung das Oxybutynin in Form des Hydrochlorids enthält, die oxybutyninhaltige Zusammensetzung als wässrige Zusammensetzung ausgebildet ist und die oxybutyninhaltige Zusammensetzung in Kombination und jeweils in wirksamen, insbesondere pharmazeutisch wirksamen Mengen,
- Oxybutyninhydrochlorid (Oxybutynin-HCI) in einer Konzentration von etwa 1 mg/ml und
- Natriumchlorid, berechnet als Natrium, in einer Konzentration von etwa 3,5 mg/ml
enthält,
wobei die oxybutyninhaltige Zusammensetzung auf einen pH-Wert im Bereich von 3,8 bis 4,5, eingestellt ist und/oder wobei die oxybutyninhaltige Zusammensetzung einen pH-Wert im Bereich von 3,8 bis 4,5 aufweist; und
wobei die in der Kolbenspritze enthaltene oxybutyninhaltige Zusammensetzung einschließlich der Kolbenspritze dampfsterilisiert, insbesondere wasserdampfsterilisiert, ist,
wobei die Dampfsterilisation unter *overkill*-Bedingungen und/oder bei etwa 121 °C und/oder für einen Zeitraum von mindestens 15 min, insbesondere von mindestens 20 min, vorzugsweise von etwa 20 min, durchgeführt wird und/oder
wobei die Dampfsterilisation gemäß Pharmacopoea Europaea (Ph. Eur.), Kapitel 5.1.1 (07/2017:50101) durchgeführt wird, insbesondere unter *overkill*-Bedingungen und/oder bei etwa 121 °C und/oder für einen Zeitraum von mindestens 15 min, insbesondere von mindestens 20 min, vorzugsweise von etwa 20 min.

Wie zuvor angeführt, betrifft die vorliegende Erfindung auch die erfindungsgemäße sterile oxybutyninhaltige Zusammensetzung zur Verwendung bei der prophylaktischen und/oder therapeutischen Behandlung von neurogenen Blasenfunktionsstörungen, insbesondere neurogenen Blasenentleerungsstörungen, vorzugsweise von mit einer Detrusorhyperaktivität und/oder einer Detrusor-Sphinkter-Dyssynergie einhergehenden neurogenen Blasenfunktionsstörungen. In diesem Zusammenhang können die neurogenen Blasenfunktionsstörungen mit einer Detrusorüberaktivität und/oder Detrusor-Sphinkter-Dyssynergie, insbesondere mit überaktivem Detrusor, einhergehen bzw. hierdurch gekennzeichnet sind. In diesem Zusammenhang können zudem die neurogenen Blasenfunktionsstörungen mit einer Rückenmarksverletzung, insbesondere einer Querschnittlähmung, Spina bifida, Diabetes, Multiple Sklerose, Schlaganfall oder Morbus Parkinson im Zusammenhang stehen oder hierdurch verursacht sein.

Insbesondere kann die erfindungsgemäße Zusammensetzung zur Instillation und/oder zur vorzugsweise topischen Applikation in den Urogenitalbereich, insbesondere in die Harnblase vorgesehen oder hergerichtet sein.

Zudem kann die oxybutyninhaltige Zusammensetzung nach der Erfindung zur Verabreichung und/oder zu Instillation und/oder zur vorzugsweise topischen Applikation in den Urogenitaltrakt, insbesondere in die Harnblase, vorgesehen bzw. hergerichtet sein.

Insbesondere kann die oxybutyninhaltige Zusammensetzung mittels Instillation und/oder mittels topischer Applikation in den Urogenitaltrakt, insbesondere in die Harnblase, verabreicht werden.

In Hinblick auf weitere Ausgestaltungen der erfindungsgemäßen Zusammensetzung kann auch auf die Ausführungen zu den weiteren erfindungsgemäßen Aspekten verwiesen werden, welche vorliegend entsprechend gelten.

Darüber hinaus ist Gegenstand der vorliegenden Erfindung - gemäß einem **weiteren** Aspekt der vorliegenden Erfindung - zudem die erfindungsgemäße Kolbenspritze, insbesondere Einweg-Kolbenspritze, wobei die Kolbenspritze einen Spritzenkörper, insbesondere Spritzenzylinder, einerseits und einen Spritzenkolben mit einem Kolbenstopfen andererseits aufweist, wobei der Spritzenkörper aus einem Cycloolefin-(Co-)Polymer (COP bzw. COC) gebildet ist und wobei der Kolbenstopfen aus einem Halogenbutylkautschuk gebildet ist,
wobei die Kolbenspritze mit einer sterilen oxybutyninhaltigen Zusammensetzung, insbesondere zur Verwendung bei der prophylaktischen und/oder therapeutischen Behandlung von neurogenen Blasenfunktionsstörungen, insbesondere neurogenen Blasenentleerungsstörungen, vorzugsweise von mit einer Detrusorhyperaktivität und/oder einer Detrusor-Sphinkter-Dyssynergie einhergehenden neurogenen Blasenfunktionsstörungen, insbesondere wie zuvor definiert, befüllt ist,
wobei die Kolbenspritze nach einem wie zuvor definierten Verfahren erhältlich ist; und/oder
wobei die in der Kolbenspritze enthaltene oxybutyninhaltige Zusammensetzung einschließlich der Kolbenspritze nach einem wie zuvor beschriebenen Verfahren dampfsterilisiert, insbesondere wasserdampfsterilisiert, worden ist.

Insbesondere betrifft die vorliegende Erfindung gemäß diesem Aspekt die erfindungsgemäße Kolbenspritze, insbesondere Einweg-Kolbenspritze, wobei die Kolbenspritze einen Spritzenkörper, insbesondere Spritzenzylinder, einerseits und einen Spritzenkolben mit einem Kolbenstopfen andererseits aufweist, wobei der Spritzenkörper aus einem Cycloolefin-(Co-)Polymer (COP bzw. COC) gebildet ist und wobei der Kolbenstopfen aus einem Halogenbutylkautschuk gebildet ist, insbesondere eine wie zuvor definierte Kolbenspritze,
wobei die Kolbenspritze mit einer sterilen oxybutyninhaltigen Zusammensetzung, insbesondere zur Verwendung bei der prophylaktischen und/oder therapeutischen Behandlung von neurogenen Blasenfunktionsstörungen, insbesondere neurogenen Blasenentleerungsstörungen, vorzugsweise von mit einer Detrusorhyperaktivität und/oder einer Detrusor-Sphinkter-Dyssynergie einhergehenden neurogenen Blasenfunktionsstörungen, befüllt ist,
wobei die oxybutyninhaltige Zusammensetzung das Oxybutynin in Form des Hydrochlorids enthält, die oxybutyninhaltige Zusammensetzung als wässrige Zusammensetzung ausgebildet ist und die oxybutyninhaltige Zusammensetzung in Kombination und jeweils in wirksamen, insbesondere pharmazeutisch wirksamen Mengen,
- Oxybutyninhydrochlorid (Oxybutynin-HCI) in einer Konzentration von (1 ± 0,5) mg/ml und
- Natriumchlorid, berechnet als Natrium, in einer Konzentration von (3,5 ± 0,5) mg/ml
enthält,
wobei die oxybutyninhaltige Zusammensetzung auf einen pH-Wert im Bereich von 3 bis 5, insbesondere im Bereich von 3,8 bis 4,5, eingestellt ist und/oder wobei die oxybutyninhaltige Zusammensetzung einen pH-Wert im Bereich von 3 bis 5, insbesondere im Bereich von 3,8 bis 4,5, aufweist; und
wobei die in der Kolbenspritze enthaltene oxybutyninhaltige Zusammensetzung einschließlich der Kolbenspritze dampfsterilisiert, insbesondere wasserdampfsterilisiert, ist.

Im Allgemeinen kann die oxybutyninhaltige Zusammensetzung das Oxybutyninhydrochlorid (Oxybutynin-HCI) in einer Konzentration von (1 ± 0,4) mg/ml, insbesondere in einer Konzentration von (1 ± 0,3) mg/ml, vorzugsweise in einer Konzentration von (1 ± 0,2) mg/ml, bevorzugt in einer Konzentration von (1 ± 0,1) mg/ml, besonders bevorzugt in einer Konzentration von etwa 1 mg/ml, enthalten.

Zudem kann die oxybutyninhaltige Zusammensetzung das Natriumchlorid, berechnet als Natrium, in einer Konzentration von (3,5 ± 0,4) mg/ml, insbesondere in einer Konzentration von (3,5 ± 0,3) mg/ml, vorzugsweise in einer Konzentration von (3,5 ± 0,2) mg/ml, bevorzugt in einer Konzentration von (3,5 ± 0,1) mg/ml, besonders bevorzugt in einer Konzentration von etwa 3,5 mg/ml, enthalten. Insbesondere kann die oxybutyninhaltige Zusammensetzung das Natriumchlorid, berechnet als Natrium, in einer Konzentration von 3,5 mg enthalten.

Erfindungsgemäß kann die Kolbenspritze ein Volumen, insbesondere ein Aufnahmevolumen für die oxybutyninhaltige Zusammensetzung, im Bereich von 4 ml bis 25 ml, insbesondere im Bereich von 6 ml bis 20 ml, vorzugsweise im Bereich von 8 ml bis 15 ml, bevorzugt im Bereich von 9 ml bis 12 ml, besonders bevorzugt etwa 10 ml, aufweisen. Insbesondere kann die Kolbenspritze ein Volumen von 10 ml aufweisen.

Gleichermaßen kann die Kolbenspritze die oxybutyninhaltige Zusammensetzung in einer Menge (Volumen) im Bereich von 4 ml bis 25 ml, insbesondere im Bereich von 6 ml bis 20 ml, vorzugsweise im Bereich von 8 ml bis 15 ml, bevorzugt im Bereich von 9 ml bis 12 ml, besonders bevorzugt von etwa 10 ml, aufweisen. Insbesondere kann die Kolbenspritze die oxybutyninhaltige Zusammensetzung in einer Menge von 10 ml aufweisen.

Erfindungsgemäß ist es bevorzugt, dass der Kolbenstopfen aus einem Chlorbutylkautschuk oder aus einem Brombutylkautschuk, vorzugsweise aus einem Brombutylkautschuk, gebildet ist.

Die erfindungsgemäße Kolbenspritze, insbesondere der Spritzenzylinder, kann im Allgemeinen eine erste Öffnung, insbesondere zur Aufnahme eines Spritzenkolbens, aufweisen.

Erfindungsgemäß kann es insbesondere vorgesehen sein, dass die Kolbenspritze einen Spritzenkolben aufweist. In diesem Zusammenhang ist es erfindungsgemäß bevorzugt, dass der Spritzenkolben aus Polypropylen gebildet ist oder dass der Spritzenkolben aus einem Cycloolefin-(Co-)Polymer (COP bzw. COC) gebildet ist.

Im Allgemeinen kann die Kolbenspritze eine zweite Öffnung, insbesondere Austragsöffnung, vorzugsweise zum Austragen bzw. Applizieren der oxybutyninhaltigen Zusammensetzung, aufweisen. Diesbezüglich kann die Kolbenspritze ein Verschlusselement, insbesondere eine Verschlusskappe, vorzugsweise zum Verschließen der zweite Öffnung, aufweisen. Erfindungsgemäß ist es dabei besonders bevorzugt, dass das Verschlusselement aus einem Halogenbutylkautschuk, insbesondere aus einem Chlorbutylkautschuk oder aus einem Brombutylkautschuk, vorzugsweise aus einem Brombutylkautschuk, gebildet ist.

Erfindungsgemäß kann die Dampfsterilisation unter mindestens einer bzw. einem der zuvor für das erfindungsgemäße Verfahren definierten (Dampfsterilisations-) Bedingungen bzw. Parameter durchgeführt sein.

Im Hinblick auf die erfindungsgemäße Kolbenspritze kann die Dampfsterilisation insbesondere unter *overkill*-Bedingungen und/oder bei etwa 121 °C und/oder für einen Zeitraum von mindestens 15 min, insbesondere von mindestens 20 min, vorzugsweise von etwa 20 min, durchgeführt sein.

Insbesondere kann die Dampfsterilisation gemäß Pharmacopoea Europaea (Ph. Eur.), Kapitel 5.1.1 (07/2017:50101) durchgeführt sein, insbesondere unter *overkill*-Bedingungen und/oder bei etwa 121 °C und/oder für einen Zeitraum von mindestens 15 min, insbesondere von mindestens 20 min, vorzugsweise von etwa 20 min.

Was die erfindungsgemäße Kolbenspritze weiterhin anbelangt, so kann die mit der nach Dampfsterilisation erhaltene sterile oxybutyninhaltige Zusammensetzung befüllte nach Dampfsterilisation erhaltene sterile Kolbenspritze und/oder die oxybutyninhaltige Zusammensetzung nach Dampfsterilisation einen SAL-Wert (*Sterility* Assurance) von mindestens 10⁻⁵, insbesondere mindestens 10⁻⁶, vorzugsweise mindestens 10⁻⁷, aufweisen.

Die sterile Kolbenspritze kann insbesondere zur Instillation und/oder zur vorzugsweise topischen Applikation der oxybutyninhaltigen Zusammensetzung in den Urogenitalbereich, insbesondere in die Harnblase eingesetzt bzw. verwendet werden bzw. hergerichtet sein.

Gemäß dem vorliegenden Aspekt betrifft die vorliegende Erfindung auch eine erfindungsgemäße Kolbenspritze, insbesondere Einweg-Kolbenspritze, wobei die Kolbenspritze einen Spritzenkörper, insbesondere Spritzenzylinder, einerseits und einen Spritzenkolben mit einem Kolbenstopfen andererseits aufweist, wobei der Spritzenkörper aus einem Cycloolefin-(Co-)Polymer (COP bzw. COC) gebildet ist und wobei der Kolbenstopfen aus einem Halogenbutylkautschuk, insbesondere aus einem Chlorbutylkautschuk oder aus einem Brombutylkautschuk, vorzugsweise aus einem Brombutylkautschuk, gebildet ist, insbesondere eine wie zuvor definierte Kolbenspritze,
wobei die Kolbenspritze mit einer sterilen oxybutyninhaltigen Zusammensetzung, insbesondere zur Verwendung bei der prophylaktischen und/oder therapeutischen Behandlung von neurogenen Blasenfunktionsstörungen, insbesondere neurogenen Blasenentleerungsstörungen, vorzugsweise von mit einer Detrusorhyperaktivität und/oder einer Detrusor-Sphinkter-Dyssynergie einhergehenden neurogenen Blasenfunktionsstörungen, befüllt ist,
wobei die oxybutyninhaltige Zusammensetzung das Oxybutynin in Form des Hydrochlorids enthält, die oxybutyninhaltige Zusammensetzung als wässrige Zusammensetzung ausgebildet ist und die oxybutyninhaltige Zusammensetzung in Kombination und jeweils in wirksamen, insbesondere pharmazeutisch wirksamen Mengen,
- Oxybutyninhydrochlorid (Oxybutynin-HCI) in einer Konzentration von (1 ± 0,4) mg/ml, insbesondere in einer Konzentration von (1 ± 0,3) mg/ml, vorzugsweise in einer Konzentration von (1 ± 0,2) mg/ml, bevorzugt in einer Konzentration von (1 ± 0,1) mg/ml, besonders bevorzugt in einer Konzentration von etwa 1 mg/ml, und
- Natriumchlorid, berechnet als Natrium, in einer Konzentration von (3,5 ± 0,4) mg/ml, insbesondere in einer Konzentration von (3,5 ± 0,3) mg/ml, vorzugsweise in einer Konzentration von (3,5 ± 0,2) mg/ml, bevorzugt in einer Konzentration von (3,5 ± 0,1) mg/ml, besonders bevorzugt in einer Konzentration von etwa 3,5 mg/ml,
enthält,
wobei die oxybutyninhaltige Zusammensetzung auf einen pH-Wert im Bereich von 3 bis 5, insbesondere im Bereich von 3,8 bis 4,5, eingestellt ist und/oder wobei die oxybutyninhaltige Zusammensetzung einen pH-Wert im Bereich von 3 bis 5, insbesondere im Bereich von 3,8 bis 4,5, aufweist; und
wobei die in der Kolbenspritze enthaltene oxybutyninhaltige Zusammensetzung einschließlich der Kolbenspritze dampfsterilisiert, insbesondere wasserdampfsterilisiert, ist.

Gemäß dem vorliegenden Aspekt betrifft die vorliegende Erfindung gleichermaßen auch eine erfindungsgemäße Kolbenspritze, insbesondere Einweg-Kolbenspritze, wobei die Kolbenspritze einen Spritzenkörper, insbesondere Spritzenzylinder, einerseits und einen Spritzenkolben mit einem Kolbenstopfen andererseits aufweist, wobei der Spritzenkörper aus einem Cycloolefin-(Co-)Polymer (COP bzw. COC) gebildet ist und wobei der Kolbenstopfen aus einem Halogenbutylkautschuk, insbesondere aus einem Chlorbutylkautschuk oder aus einem Brombutylkautschuk, vorzugsweise aus einem Brombutylkautschuk, gebildet ist, insbesondere eine wie zuvor definierte Kolbenspritze,
wobei die Kolbenspritze mit einer sterilen oxybutyninhaltigen Zusammensetzung, insbesondere zur Verwendung bei der prophylaktischen und/oder therapeutischen Behandlung von neurogenen Blasenfunktionsstörungen, insbesondere neurogenen Blasenentleerungsstörungen, vorzugsweise von mit einer Detrusorhyperaktivität und/oder einer Detrusor-Sphinkter-Dyssynergie einhergehenden neurogenen Blasenfunktionsstörungen, befüllt ist,
wobei die oxybutyninhaltige Zusammensetzung das Oxybutynin in Form des Hydrochlorids enthält, die oxybutyninhaltige Zusammensetzung als wässrige Zusammensetzung ausgebildet ist und die oxybutyninhaltige Zusammensetzung in Kombination und jeweils in wirksamen, insbesondere pharmazeutisch wirksamen Mengen,
- Oxybutyninhydrochlorid (Oxybutynin-HCI) in einer Konzentration von (1 ± 0,4) mg/ml, insbesondere in einer Konzentration von (1 ± 0,3) mg/ml, vorzugsweise in einer Konzentration von (1 ± 0,2) mg/ml, bevorzugt in einer Konzentration von (1 ± 0,1) mg/ml, besonders bevorzugt in einer Konzentration von etwa 1 mg/ml, und
- Natriumchlorid, berechnet als Natrium, in einer Konzentration von (3,5 ± 0,4) mg/ml, insbesondere in einer Konzentration von (3,5 ± 0,3) mg/ml, vorzugsweise in einer Konzentration von (3,5 ± 0,2) mg/ml, bevorzugt in einer Konzentration von (3,5 ± 0,1) mg/ml, besonders bevorzugt in einer Konzentration von etwa 3,5 mg/ml,
enthält,
wobei die oxybutyninhaltige Zusammensetzung auf einen pH-Wert im Bereich von 3 bis 5, insbesondere im Bereich von 3,8 bis 4,5, eingestellt ist und/oder wobei die oxybutyninhaltige Zusammensetzung einen pH-Wert im Bereich von 3 bis 5, insbesondere im Bereich von 3,8 bis 4,5, aufweist; und
wobei die in der Kolbenspritze enthaltene oxybutyninhaltige Zusammensetzung einschließlich der Kolbenspritze dampfsterilisiert, insbesondere wasserdampfsterilisiert, ist.

Der vorliegende Aspekt umfasst gleichermaßen auch eine erfindungsgemäße Kolbenspritze, insbesondere Einweg-Kolbenspritze, wobei die Kolbenspritze einen Spritzenkörper, insbesondere Spritzenzylinder, einerseits und einen Spritzenkolben mit einem Kolbenstopfen andererseits aufweist, wobei der Spritzenkörper aus einem Cycloolefin-(Co-)Polymer (COP bzw. COC) gebildet ist und wobei der Kolbenstopfen aus einem Halogenbutylkautschuk, insbesondere aus einem Chlorbutylkautschuk oder aus einem Brombutylkautschuk, vorzugsweise aus einem Brombutylkautschuk, gebildet ist, insbesondere eine wie zuvor definierte Kolbenspritze,
wobei die Kolbenspritze mit einer sterilen oxybutyninhaltigen Zusammensetzung, insbesondere zur Verwendung bei der prophylaktischen und/oder therapeutischen Behandlung von neurogenen Blasenfunktionsstörungen, insbesondere neurogenen Blasenentleerungsstörungen, vorzugsweise von mit einer Detrusorhyperaktivität und/oder einer Detrusor-Sphinkter-Dyssynergie einhergehenden neurogenen Blasenfunktionsstörungen, befüllt ist,
wobei die oxybutyninhaltige Zusammensetzung das Oxybutynin in Form des Hydrochlorids enthält, die oxybutyninhaltige Zusammensetzung als wässrige Zusammensetzung ausgebildet ist und die oxybutyninhaltige Zusammensetzung in Kombination und jeweils in wirksamen, insbesondere pharmazeutisch wirksamen Mengen,
- Oxybutyninhydrochlorid (Oxybutynin-HCI) in einer Konzentration von etwa 1 mg/ml und
- Natriumchlorid, berechnet als Natrium, in einer Konzentration von etwa 3,5 mg/ml
enthält,
wobei die oxybutyninhaltige Zusammensetzung auf einen pH-Wert im Bereich von 3,8 bis 4,5 eingestellt ist und/oder wobei die oxybutyninhaltige Zusammensetzung einen pH-Wert im Bereich von 3,8 bis 4,5 aufweist; und
wobei die in der Kolbenspritze enthaltene oxybutyninhaltige Zusammensetzung einschließlich der Kolbenspritze dampfsterilisiert, insbesondere wasserdampfsterilisiert, ist.

Gemäß dem vorliegenden Aspekt betrifft die vorliegende Erfindung auch eine Kolbenspritze, insbesondere Einweg-Kolbenspritze, wobei die Kolbenspritze einen Spritzenkörper, insbesondere Spritzenzylinder, einerseits und einen Spritzenkolben mit einem Kolbenstopfen andererseits aufweist, wobei der Spritzenkörper aus einem Cycloolefin-(Co-)Polymer (COP bzw. COC) gebildet ist und wobei der Kolbenstopfen aus einem Halogenbutylkautschuk, insbesondere aus einem Chlorbutylkautschuk oder aus einem Brombutylkautschuk, vorzugsweise aus einem Brombutylkautschuk, gebildet ist, insbesondere eine wie zuvor definierte Kolbenspritze,
wobei die Kolbenspritze mit einer sterilen oxybutyninhaltigen Zusammensetzung, insbesondere zur Verwendung bei der prophylaktischen und/oder therapeutischen Behandlung von neurogenen Blasenfunktionsstörungen, insbesondere neurogenen Blasenentleerungsstörungen, vorzugsweise von mit einer Detrusorhyperaktivität und/oder einer Detrusor-Sphinkter-Dyssynergie einhergehenden neurogenen Blasenfunktionsstörungen, befüllt ist,
wobei die oxybutyninhaltige Zusammensetzung das Oxybutynin in Form des Hydrochlorids enthält, die oxybutyninhaltige Zusammensetzung als wässrige Zusammensetzung ausgebildet ist und die oxybutyninhaltige Zusammensetzung in Kombination und jeweils in wirksamen, insbesondere pharmazeutisch wirksamen Mengen,
- Oxybutyninhydrochlorid (Oxybutynin-HCI) in einer Konzentration von etwa 1 mg/ml und
- Natriumchlorid, berechnet als Natrium, in einer Konzentration von etwa 3,5 mg/ml
enthält,
wobei die oxybutyninhaltige Zusammensetzung auf einen pH-Wert im Bereich von 3,8 bis 4,5 eingestellt ist und/oder wobei die oxybutyninhaltige Zusammensetzung einen pH-Wert im Bereich von 3,8 bis 4,5 aufweist; und
wobei die in der Kolbenspritze enthaltene oxybutyninhaltige Zusammensetzung einschließlich der Kolbenspritze dampfsterilisiert, insbesondere wasserdampfsterilisiert, ist,
wobei die Dampfsterilisation unter *overkill*-Bedingungen und/oder bei etwa 121 °C und/oder für einen Zeitraum von mindestens 15 min, insbesondere von mindestens 20 min, vorzugsweise von etwa 20 min, durchgeführt wird und/oder
wobei die Dampfsterilisation gemäß Pharmacopoea Europaea (Ph. Eur.), Kapitel 5.1.1 (07/2017:50101) durchgeführt wird, insbesondere unter *overkill*-Bedingungen und/oder bei etwa 121 °C und/oder für einen Zeitraum von mindestens 15 min, insbesondere von mindestens 20 min, vorzugsweise von etwa 20 min.

Die vorliegende Erfindung betrifft gemäß dem vorliegenden Aspekt auch eine Kolbenspritze, insbesondere Einweg-Kolbenspritze, wobei die Kolbenspritze einen Spritzenkörper, insbesondere Spritzenzylinder, einerseits und einen Spritzenkolben mit einem Kolbenstopfen andererseits aufweist, wobei der Spritzenkörper aus einem Cycloolefin-(Co-)Polymer (COP bzw. COC) gebildet ist und wobei der Kolbenstopfen aus einem Halogenbutylkautschuk, insbesondere aus einem Chlorbutylkautschuk oder aus einem Brombutylkautschuk, vorzugsweise aus einem Brombutylkautschuk, gebildet ist, insbesondere eine zuvor definierte Kolbenspritze,
wobei die Kolbenspritze mit einer sterilen oxybutyninhaltigen Zusammensetzung, insbesondere zur Verwendung bei der prophylaktischen und/oder therapeutischen Behandlung von neurogenen Blasenfunktionsstörungen, insbesondere neurogenen Blasenentleerungsstörungen, vorzugsweise von mit einer Detrusorhyperaktivität und/oder einer Detrusor-Sphinkter-Dyssynergie einhergehenden neurogenen Blasenfunktionsstörungen, befüllt ist,
wobei die oxybutyninhaltige Zusammensetzung das Oxybutynin in Form des Hydrochlorids enthält, die oxybutyninhaltige Zusammensetzung als wässrige Zusammensetzung ausgebildet ist und die oxybutyninhaltige Zusammensetzung in Kombination und jeweils in wirksamen, insbesondere pharmazeutisch wirksamen Mengen,
- Oxybutyninhydrochlorid (Oxybutynin-HCI) in einer Konzentration von etwa 1 mg/ml und
- Natriumchlorid, berechnet als Natrium, in einer Konzentration von etwa 3,5 mg/ml
enthält,
wobei die oxybutyninhaltige Zusammensetzung auf einen pH-Wert im Bereich von 3,8 bis 4,5 eingestellt ist und/oder wobei die oxybutyninhaltige Zusammensetzung einen pH-Wert im Bereich von 3,8 bis 4,5 aufweist; und
wobei die Kolbenspritze ein Volumen, insbesondere ein Aufnahmevolumen für die oxybutyninhaltige Zusammensetzung, von etwa 10 ml, aufweist und/oder wobei die Kolbenspritze die oxybutyninhaltige Zusammensetzung in einer Menge von etwa 10 ml aufweist,
wobei die in der Kolbenspritze enthaltene oxybutyninhaltige Zusammensetzung einschließlich der Kolbenspritze dampfsterilisiert, insbesondere wasserdampfsterilisiert, ist.

Im Hinblick auf weitere Ausgestaltungen der erfindungsgemäßen Kolbenspritze kann auch auf die Ausführungen zu den weiteren erfindungsgemäßen Aspekten verwiesen werden, welche vorliegend entsprechend gelten.

Die vorliegende Erfindung betrifft weiterhin - gemäß einem wiederum **weiteren** Aspekt der vorliegenden Erfindung - auch die erfindungsgemäße Verpackungseinheit, enthaltend mindestens eine Verpackung und mindestens eine Kolbenspritze, insbesondere Einweg-Kolbenspritze, wie zuvor definiert, wobei die Kolbenspritze in die Verpackung eingebracht ist bzw. in der Verpackung vorliegt.

In diesem Zusammenhang kann es sich erfindungsgemäß insbesondere derart verhalten, dass die erfindungsgemäße Verpackungseinheit bzw. die diesbezügliche Verpackung mit der diesbezüglichen Kolbenspritze und der oxybutyninhaltigen Zusammensetzung gemäß dem erfindungsgemäßen Verfahren, wie zuvor definiert, dampfsterilisiert worden ist.

Darüber hinaus kann die Verpackungseinheit im Allgemeinen als Bereitschaftspackung ausgebildet sein. Insbesondere kann die Verpackungseinheit nach der Erfindung wasser- und/oder keimdicht verschlossen bzw. versiegelt sein. Weiterhin kann zumindest ein Teil der Verpackung wenigstens wasserdampfdurchlässig, insbesondere wasserdampf- und/oder gasdurchlässig ausgebildet sein.

Für weitere Ausführungen bzw. Ausgestaltungen zu der erfindungsgemäßen Verpackungseinheit kann zudem auf die weiteren Ausführungen zu den weiteren Aspekten der vorliegenden Erfindung verwiesen werden, welche entsprechend gelten.

Wiederum weiterer Gegenstand der vorliegenden Erfindung - gemäß einem **weiteren** Aspekt der vorliegenden Erfindung - ist zudem das erfindungsgemäße Kit, insbesondere Applikations- und/oder Instillationssystem, umfassend (i) mindestens eine in einer Kolbenspritze, insbesondere Einweg-Kolbenspritze, insbesondere wie zuvor definiert, vorliegende Zusammensetzung, wie zuvor definiert; (ii) (a) mindestens eine an die Kolbenspritze anschließbare Applikations- und/oder Instillationsvorrichtung, insbesondere in Form eines Instillationsschlauches oder dergleichen, vorzugsweise in Form eines (Harn-blasen-)Katheters, und/oder (b) mindestens eine an die Kolbenspritze anschließbare Anschlussvorrichtung, insbesondere Adapter, zum Anschließen an eine Applikations- und/oder Instillationsvorrichtung, insbesondere in Form eines Instillationsschlauches oder dergleichen, vorzugsweise in Form eines (Harnblasen-)Katheters; und gegebenenfalls (iii) mindestens eine Applikations- und/oder Instillationsanleitung.

Gemäß diesem Aspekt betrifft die vorliegende Erfindung auch das erfindungsgemäße Kit, insbesondere Applikations- und/oder Instillationssystem, umfassend (i) mindestens eine Kolbenspritze, insbesondere Einweg-Kolbenspritze, wie zuvor definiert, und/oder mindestens eine Verpackungseinheit, wie zuvor definiert; (ii) (a) mindestens eine an die Kolbenspritze anschließbare Applikations- und/oder Instillationsvorrichtung, insbesondere in Form eines Instillationsschlauches oder dergleichen, vorzugsweise in Form eines (Harn-blasen)Katheters, und/oder (b) mindestens eine an die Kolbenspritze anschließbare Anschlussvorrichtung, insbesondere Adapter, zum Anschließen an eine Applikations- und/oder Instillationsvorrichtung, insbesondere in Form eines Instillationsschlauches oder dergleichen, vorzugsweise in Form eines (Harnblasen-)Katheters; und gegebenenfalls (iii) mindestens eine Applikations- und/oder Instillationsanleitung.

Hinsichtlich weiterer diesbezüglicher Ausgestaltungen kann zudem auf die Ausführungen zu den weiteren erfindungsgemäßen Aspekten verwiesen werden, welche entsprechend gelten.

Nochmals weiterer Gegenstand der vorliegenden Erfindung - gemäß einem **weiteren** Aspekt der vorliegenden Erfindung - ist zudem die erfindungsgemäße Verwendung einer Kolbenspritze, insbesondere Einweg-Kolbenspritze, in einem Verfahren zur Herstellung einer sterilen oxybutyninhaltigen Zusammensetzung, insbesondere wie zuvor definiert,
wobei die Kolbenspritze einen Spritzenkörper, insbesondere Spritzenzylinder, einerseits und einen Spritzenkolben mit einem Kolbenstopfen andererseits aufweist, wobei der Spritzenkörper aus einem Cycloolefin-(Co-)Polymer (COP bzw. COC) gebildet ist und wobei der Kolbenstopfen aus einem Halogenbutylkautschuk gebildet ist,
wobei die Kolbenspritze mit der sterilen oxybutyninhaltigen Zusammensetzung, insbesondere zur Verwendung bei der prophylaktischen und/oder therapeutischen Behandlung von neurogenen Blasenfunktionsstörungen, insbesondere neurogenen Blasenentleerungsstörungen, vorzugsweise von mit einer Detrusorhyperaktivität und/oder einer Detrusor-Sphinkter-Dyssynergie einhergehenden neurogenen Blasenfunktionsstörungen, befüllt ist,
wobei die oxybutyninhaltige Zusammensetzung das Oxybutynin in Form des Hydrochlorids enthält, die oxybutyninhaltige Zusammensetzung als wässrige Zusammensetzung ausgebildet ist und die oxybutyninhaltige Zusammensetzung in Kombination und jeweils in wirksamen, insbesondere pharmazeutisch wirksamen Mengen,
- Oxybutyninhydrochlorid (Oxybutynin-HCI) in einer Konzentration von (1 ± 0,5) mg/ml und
- Natriumchlorid, berechnet als Natrium, in einer Konzentration von (3,5 ± 0,5) mg/ml
enthält,
wobei die oxybutyninhaltige Zusammensetzung auf einen pH-Wert im Bereich von 3 bis 5, insbesondere im Bereich von 3,8 bis 4,5, eingestellt ist und/oder wobei die oxybutyninhaltige Zusammensetzung einen pH-Wert im Bereich von 3 bis 5, insbesondere im Bereich von 3,8 bis 4,5, aufweist; und
wobei die in der Kolbenspritze enthaltene oxybutyninhaltige Zusammensetzung einschließlich der Kolbenspritze dampfsterilisiert, insbesondere wasserdampfsterilisiert, wird.

Diesbezüglich kann auch auf die Ausführungen zu den weiteren erfindungsgemäßen Aspekten gelten, welche vorliegend entsprechend gelten.

Schließlich ist ein weiterer Gegenstand der vorliegenden Erfindung - gemäß einem **weiteren** Aspekt der vorliegenden Erfindung - zudem die erfindungsgemäße Verwendung einer Dampfsterilisation, insbesondere Wasserdampfsterilisation, in einem Verfahren zur Herstellung einer sterilen oxybutyninhaltigen Zusammensetzung in einer vorzugsweise anwendungsfertigen Kolbenspritze, insbesondere Einweg-Kolbenspritze, und/oder zur Herstellung einer vorzugsweise anwendungsfertigen, sterilen, mit einer oxybutyninhaltigen Zusammensetzung befüllten Kolbenspritze, insbesondere Einweg-Kolbenspritze, insbesondere wie zuvor definiert,
wobei die Kolbenspritze einen Spritzenkörper, insbesondere Spritzenzylinder, einerseits und einen Spritzenkolben mit einem Kolbenstopfen andererseits aufweist, wobei der Spritzenkörper aus einem Cycloolefin-(Co-)Polymer (COP bzw. COC) gebildet ist und wobei der Kolbenstopfen aus einem Halogenbutylkautschuk gebildet ist;
wobei in der oxybutyninhaltigen Zusammensetzung das Oxybutynin in Form des Hydrochlorids eingesetzt wird, die oxybutyninhaltige Zusammensetzung als wässrige Zusammensetzung ausgebildet wird und die oxybutyninhaltige Zusammensetzung in Kombination und jeweils in wirksamen, insbesondere pharmazeutisch wirksamen Mengen,
- Oxybutyninhydrochlorid (Oxybutynin-HCI) in einer Konzentration von (1 ± 0,5) mg/ml und
- Natriumchlorid, berechnet als Natrium, in einer Konzentration von (3,5 ± 0,5) mg/ml
enthält,
wobei die oxybutyninhaltige Zusammensetzung auf einen pH-Wert im Bereich von 3 bis 5, insbesondere im Bereich von 3,8 bis 4,5, eingestellt wird und/oder wobei die oxybutyninhaltige Zusammensetzung einen pH-Wert im Bereich von 3 bis 5, insbesondere im Bereich von 3,8 bis 4,5, aufweist; und
wobei die mit der oxybutyninhaltigen Zusammensetzung befüllte Kolbenspritze und/oder die in der Kolbenspritze bereitgestellte oxybutyninhaltige Zusammensetzung dampfsterilisiert, insbesondere wasserdampfsterilisiert, wird.

Für weitere diesbezügliche Ausführungen zu der erfindungsgemäßen Verwendung gemäß diesem Aspekt kann auch auf die Ausführungen zu den weiteren erfindungsgemäßen Aspekten verwiesen werden, welche vorliegend entsprechend gelten.

Die vorliegende Erfindung wird nachfolgend auch anhand einer bevorzugte Ausführungsformen bzw. Ausgestaltungen darstellenden Figurendarstellung beschrieben. Im Zusammenhang mit der Erläuterung dieser bevorzugten Ausführungsform bzw. Ausgestaltung der vorliegenden Erfindung, welche jedoch in Bezug auf die vorliegende Erfindung keinesfalls beschränkend ist, werden auch weitergehende Vorteile, Eigenschaften, Aspekte und Merkmale der vorliegenden Erfindung aufgezeigt.

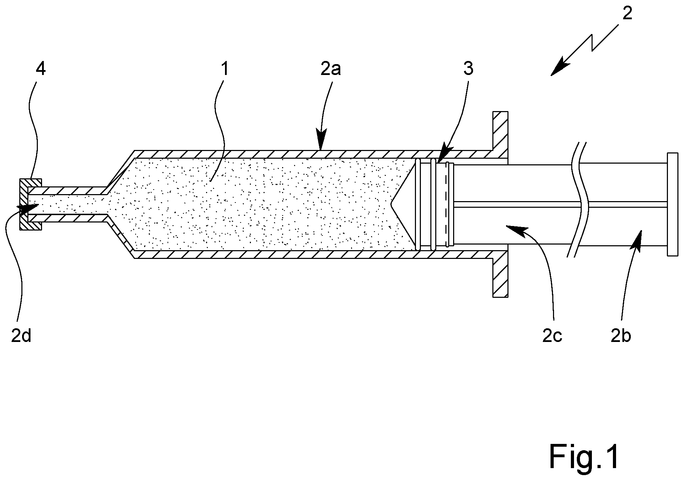

Die Figurendarstellung gemäß
- Fig. 1: zeigt dabei eine schematische Schnittdarstellung einer im Rahmen des erfindungsgemäßen Verfahren verwendeten Kolbenspritze bzw. eine mit der sterilen oxybutyninhaltigen Zusammensetzung befüllte Kolbenspritze als solche nach der Erfindung in der Seitenansicht.

Insbesondere zeigt Fig. 1 auch die sterile oxybutyninhaltige Zusammensetzung 1 nach der Erfindung, mit welcher die erfindungsgemäße Kolbenspritze 2 befüllt ist, wobei die Kolbenspritze 2 insbesondere als Einweg-Kolbenspritze ausgebildet ist und wobei die Kolbenspritze 2 einen Spritzenkörper 2a, insbesondere Spritzenzylinder, einerseits und einen Spritzenkolben 2b mit einem Kolbenstopfen 3 andererseits aufweist. Der Spritzenkörper 2a ist dabei aus einem Cycloolefin-(CO-)Polymer (COP bzw. COC) gebildet, und der Kolbenstopfen 3 ist aus einem Halogenbutylkautschuk, insbesondere aus einem Chlorbutylkautschuk oder aus einem Brombutylkautschuk, vorzugsweise aus einem Brombutylkautschuk, gebildet. Fig. 1 zeigt, dass die Kolbenspritze 2 mit der sterilen oxybutyninhaltigen Zusammensetzung 1 nach der vorliegenden Erfindung befüllt ist. Fig. 1 verdeutlicht weiterhin, dass die erfindungsgemäße Kolbenspritze 2 eine erste Öffnung 2c zur Aufnahme des Spritzenkolbens 2b mit dem Kolbenstopfen 3 aufweist und dass die erfindungsgemäße Kolbenspritze 2 zudem insbesondere gegenüberliegend zu der ersten Öffnung 2c eine zweite Öffnung 2d, insbesondere Austragsöffnung, aufweist. Die zweite Öffnung 2d ist mit einem Verschlusselement 4 in Form einer Verschlusskappe verschlossen, wobei das Verschlusselement bzw. die Verschlusskappe aus einem Halogenbutylkautschuk, insbesondere aus einem Chlorbutylkautschuk oder aus einem Brombutylkautschuk, vorzugsweise aus einem Brombutylkautschuk, gebildet ist. Fig. 1 zeigt somit den verschlossenen Zustand der Kolbenspritze mit der darin befindlichen oxybutyninhaltigen Zusammensetzung nach der Erfindung. Im Rahmen des erfindungsgemäßen Verfahrens kann diese Einheit aus verschlossener Kolbenspritze mit der darin befindlichen oxybutyninhaltigen Zusammensetzung dampfsterilisiert werden, so dass auf diese Weise eine entsprechend sterile oxybutyninhaltige Zusammensetzung bzw. eine sterile und mit der Zusammensetzung befüllte Kolbenspritze nach der Erfindung erhalten wird.

Weitere Ausgestaltungen, Abwandlungen und Variationen sowie Vorteile der vorliegenden Erfindung sind für den Fachmann beim Lesen der Beschreibung ohne Weiteres erkennbar und realisierbar, ohne dass er dabei den Rahmen der vorliegenden Erfindung verlässt.

Die folgenden Ausführungsbeispiele dienen lediglich der Veranschaulichung der vorliegenden Erfindung, jedoch ohne die vorliegende Erfindung hierauf zu beschränken.

### AUSFÜHRUNGSBEISPIELE:

Die nachfolgend beschriebenen Ausführungen zeigen die verbesserten Eigenschaften der auf Basis des erfindungsgemäßen Verfahrens bereitgestellten sterilen oxybutyninhaltigen Zusammensetzung, und zwar was die verbesserte Stabilität der Zusammensetzung bei der Sterilisation mit der Verringerung von Abbauprodukten von Oxybutyninhydrochlorid anbelangt.

Insbesondere verhält es sich bei einer terminalen Dampfsterilisation einer Zusammensetzung zur Instillation in die Blase, welche den Wirkstoff Oxybutyninhydrochlorid in einer Konzentration von 1 mg/ml bei einem pH-Wert der Zusammensetzung von 4,0, enthält und welche - im Gegensatz zur vorliegenden Erfindung - in Polypropylenspritzen mit Kolbenstopfen aus einem üblichen Gummimaterial vorgefüllt ist, derart, dass nach Durchführung einer Dampfsterilisation der Gehalt an Oxybutynin auf 85 bis 90 % des zuvor vorliegenden Wirkstoffgehalts absinkt. Dabei entsteht bei der Dampfsterilisierung in Kolbenspritzen aus Polypropylen mit Kolbenstopfen aus herkömmlichem Gummimaterial das Oxybutynin-Abbauprodukt Phenylcyclohexylglycolsäure, und zwar in solchen Mengen, welche um einen Faktor 2 bis 3 oberhalb der Spezifikationsgrenze liegen. Zudem resultieren weitere Verunreinigungen in Mengen von teilweise über 0,5 %.

Ohne sich auf diese Theorie beschränken zu wollen, liegt - im Gegensatz zu den erfindungsgemäß verwendeten Materialien für Polypropylen mitunter eine nur geringe Säurebeständigkeit vor, wobei die bei der Dampfsterilisierung vorliegenden hohen Temperaturen zudem mögliche Reaktionen zwischen der Zusammensetzung und dem Spritzenmaterial begünstigen, was auch zur Ausbildung von den weiteren Verunreinigungen führen kann. Dabei verhält es sich für das Abbauprodukt von Oxybutyninhydrochlorid in Form von Phenylcyclohexylglycolsäure insbesondere derart, dass dieses grundsätzlich durch zwei unterschiedliche Reaktionen entstehen kann, und zwar einerseits aus einer Verseifung in alkalischer Lösung oder andererseits aus einer Hydrolyse in saurer Lösung. Da der pH-Wert der Zusammensetzung im sauren Bereich liegt, kann - ohne sich auf diese Theorie beschränken zu wollen - davon ausgegangen werden, dass das Abbauprodukt vorrangig aus einer Hydrolyse von Oxybutyninhydrochlorid hervorgeht, wobei diese Reaktion durch die bei der Sterilisation vorliegenden hohen Temperaturen nochmals verstärkt wird. Durch die Hydrolyse des Wirkstoffs resultiert sowohl eine Verringerung des Wirkstoffgehalts als auch die Bildung von Phenylcyclohexylgly-colsäure bei vorliegender Dampfsterilisation. Zudem wird - im Gegensatz zur vorliegenden Erfindung - die Bildung von Phenylcyclohexylglycolsäure infolge des für den Kolbenstopfen üblicherweise verwendeten Gummimaterials weiterführend verstärkt bzw. beschleunigt. Ohne sich auf diese Theorie beschränken zu wollen, verhält es sich im Allgemeinen derart, dass Gummimaterialien, wie sie üblicherweise für Kolbenstopfen eingesetzt werden, im Allgemeinen einen hohen Anteil an ungesättigten C-C-Doppelbindungen aufweisen, was eine erhöhte Reaktivität des Materials zur Folge hat. Zudem verhält es sich - ohne sich auf diese Theorie beschränken zu wollen - für Phenylcyclohexylglycolsäure derart, dass diese durch die freie Säuregruppe reaktiver ist als die Wirksubstanz, so dass bei der Sterilisation entstehende Phenylcyclohexylglycolsäure zumindest teilweise mit dem Gummimaterial reagiert und so dem Reaktionsgleichgewicht entzogen wird, was den Abbau der Wirksubstanz weiter bevorzugt.

Im Gegensatz hierzu wird im Rahmen der vorliegenden Erfindung unter Verwendung von Kolbenspritzen auf Basis von Cycloolefin-(Co-)Polymeren (COP bzw. COC) und Kolbenstopfen bzw. Verschlusskappen auf Basis von Halogenbutylkautschuk, insbesondere Chlorbutylkautschuk oder Brombutylkautschuk, vorzugsweise Brombutylkautschuk, und einer entsprechenden oxybutyninhaltigen Zusammensetzung eine deutlich verbesserte Stabilität der Zusammensetzung mit einem geringerem Abbau der Wirksubstanz und somit einer verringerten Bildung von Abbauprodukten, insbesondere Phenylcyclohexylglycolsäure, unter definierter Dampfsterilisation beobachtet. Das erfindungsgemäß für die Kolbenspritze eingesetzte Cycloolefin-(Co-)Polymer (COP bzw. COC) weist dabei im Gegensatz zu Polypropylen eine hohe Stabilität auch gegenüber sauren Zusammensetzungen auf. Zudem verhält es sich in Bezug auf den Kolbenstopfen bzw. der Verschlusskappe derart, dass der erfindungsgemäß hierfür eingesetzte Halogenbutylkautschuk, wie zuvor angeführt, eine wesentlich geringere Anzahl an C-C-Doppelbindungen aufweist und somit weniger reaktiv ist als üblicherweise eingesetzte Gummimaterialien. Zudem weist Halogenbutylkautschuk eine verbesserte Hitzestabilität und verbesserte Alterungseigenschaften und eine geringe Gaspermeabilität auf.

Auf dieser Basis kann im Rahmen der vorliegenden Erfindung bei der Dampfsterilisation eine entsprechend sterile oxybutyninhaltige Zusammensetzung mit verbesserten Eigenschaften erhalten werden, und zwar sowohl im Hinblick auf die Gewährleistung einer hohen Sterilität als auch im Hinblick auf die Gewährleistung eines hohen bzw. unveränderten Wirkstoffgehalts, bedingt durch eine deutliche Verringerung der Bildung entsprechender Abbauprodukte.

Insgesamt ist somit festzustellen, dass auf Basis des erfindungsgemäßen Verfahrens unter Verwendung einer speziellen oxybutyninhaltigen Zusammensetzung und unter Verwendung einer speziellen Kolbenspritze mit diesbezüglich speziellen Komponenten eine effiziente und zugleich schonende Dampfsterilisation der zugrundeliegenden Zusammensetzung durchgeführt werden kann, wobei der Abbau der Wirksubstanz verringert wird, so dass auch diesbezüglich entsprechend stabile Zusammensetzungen bereitgestellt werden.

## Patentansprüche

1. Verfahren zur Herstellung einer sterilen oxybutyninhaltigen Zusammensetzung in einer vorzugsweise anwendungsfertigen Kolbenspritze, insbesondere Einweg-Kolbenspritze, und/oder zur Herstellung einer vorzugsweise anwendungsfertigen, sterilen, mit einer oxybutyninhaltigen Zusammensetzung befüllten Kolbenspritze, insbesondere Einweg-Kolbenspritze,
wobei die Kolbenspritze einen Spritzenkörper, insbesondere Spritzenzylinder, einerseits und einen Spritzenkolben mit einem Kolbenstopfen andererseits aufweist, wobei der Spritzenkörper aus einem Cycloolefin-(Co-)Polymer (COP bzw. COC) gebildet ist und wobei der Kolbenstopfen aus einem Halogenbutylkautschuk gebildet ist;
wobei das Verfahren die nachfolgenden Schritte umfasst:
a) Befüllen der Kolbenspritze mit der oxybutyninhaltigen Zusammensetzung und/oder Bereitstellen der oxybutyninhaltigen Zusammensetzung in der Kolbenspritze,
wobei das Oxybutynin in Form des Hydrochlorids eingesetzt wird, die oxybutyninhaltige Zusammensetzung als wässrige Zusammensetzung ausgebildet wird und die oxybutyninhaltige Zusammensetzung in Kombination und jeweils in wirksamen, insbesondere pharmazeutisch wirksamen Mengen,
- Oxybutyninhydrochlorid (Oxybutynin-HCI) in einer Konzentration von (1 ± 0,5) mg/ml und
- Natriumchlorid, berechnet als Natrium, in einer Konzentration von (3,5 ± 0,5) mg/ml
enthält,
wobei die oxybutyninhaltige Zusammensetzung auf einen pH-Wert im Bereich von 3 bis 5, insbesondere im Bereich von 3,8 bis 4,5, eingestellt wird und/oder wobei die oxybutyninhaltige Zusammensetzung einen pH-Wert im Bereich von 3 bis 5, insbesondere im Bereich von 3,8 bis 4,5, aufweist;
und nachfolgend
b) Dampfsterilisation, insbesondere Wasserdampfsterilisation, der oxybutyninhaltigen Zusammensetzung in der Kolbenspritze und/oder der mit der oxybutyninhaltigen Zusammensetzung befüllten Kolbenspritze.

2. Verfahren nach Anspruch 1,
wobei der Kolbenstopfen aus einem Chlorbutylkautschuk oder aus einem Brombutylkautschuk, vorzugsweise aus einem Brombutylkautschuk, gebildet ist; und/oder
wobei die Kolbenspritze, insbesondere der Spritzenzylinder, eine erste Öffnung, insbesondere zur Aufnahme eines Spritzenkolbens, aufweist; und/oder
wobei die Kolbenspritze einen Spritzenkolben aufweist, insbesondere wobei der Spritzenkolben aus Polypropylen gebildet ist oder insbesondere wobei der Spritzenkolben aus einem Cycloolefin-(Co-) Polymer (COP bzw. COC) gebildet ist; und/oder
wobei die Kolbenspritze eine zweite Öffnung, insbesondere Austragsöffnung, vorzugsweise zum Austragen und/oder Applizieren der oxybutyninhaltigen Zusammensetzung, aufweist; und/oder
wobei die Kolbenspritze ein Verschlusselement, insbesondere eine Verschlusskappe, vorzugsweise zum Verschließen der zweiten Öffnung, aufweist, insbesondere wobei das Verschlusselement aus einem Halogenbutylkautschuk, insbesondere aus einem Chlorbutylkautschuk oder aus einem Brombutylkautschuk, vorzugsweise aus einem Brombutylkautschuk, gebildet ist.

3. Verfahren nach einem der vorangehenden Ansprüche,
wobei die Dampfsterilisation unter Temperaturbeaufschlagung (Erwärmung) durchgeführt wird; und/oder
wobei die Dampfsterilisation bei Temperaturen von mindestens 105 °C, insbesondere mindestens 110 °C, vorzugsweise mindestens 115 °C, bevorzugt mindestens 120 °C, durchgeführt wird; und/oder
wobei die Dampfsterilisation bei Temperaturen von höchstens 160 °C, insbesondere höchstens 150 °C, vorzugsweise höchstens 140° C bevorzugt höchstens 130° C, durchgeführt wird; und/oder
wobei die Dampfsterilisation bei Temperaturen im Bereich von 105 °C bis 160 °C, insbesondere im Bereich von 110 °C bis 150 °C, vorzugsweise im Bereich von 115 °C bis 140 °C, bevorzugt im Bereich von 120 °C bis 130° C, besonders bevorzugt bei etwa 121 °C, durchgeführt wird; und/oder
wobei die Dampfsterilisation für einen Zeitraum von mindestens 1 min, insbesondere von mindestens 2 min, vorzugsweise von mindestens 3 min, bevorzugt von mindestens 5 min, besonders bevorzugt von mindestens 8 min, ganz besonders bevorzugt von mindestens 10 min, weiter bevorzugt von mindestens 15 min, noch weiter bevorzugt von mindestens 17 min, durchgeführt wird; und/oder
wobei die Dampfsterilisation für einen Zeitraum von höchstens 500 min, insbesondere von höchstens 400 min, vorzugsweise von höchstens 200 min, bevorzugt von höchstens 100 min, besonders bevorzugt von höchstens 60 min, ganz besonders bevorzugt von höchstens 45 min, weiter bevorzugt von höchstens 30 min, noch weiter bevorzugt von höchstens 25 min, durchgeführt wird; und/oder
wobei die Dampfsterilisation für einen Zeitraum im Bereich von 1 min bis 500 min, insbesondere im Bereich von 2 min bis 400 min, vorzugsweise im Bereich von 3 min bis 200 min, bevorzugt im Bereich von 5 min bis 100 min, besonders bevorzugt im Bereich von 8 min bis 60 min, ganz besonders bevorzugt im Bereich von 10 min bis 45 min, weiter bevorzugt im Bereich von 15 min bis 30 min, noch weiter bevorzugt im Bereich von 17 min bis 25 min, nochmals weiter bevorzugt für etwa 20 min, durchgeführt wird.

4. Verfahren nach einem der vorangehenden Ansprüche,
wobei die nach Dampfsterilisation erhaltene sterile oxybutyninhaltige Zusammensetzung und/oder die mit der oxybutyninhaltigen Zusammensetzung befüllte nach Dampfsterilisation erhaltene sterile Kolbenspritze einen SAL-Wert *(Sterility Assurance Level*) von mindestens 10⁻⁵, insbesondere mindestens 10⁻⁶, vorzugsweise mindestens 10⁻⁷, aufweist; und/oder
wobei die nach Dampfsterilisation erhaltene sterile oxybutyninhaltige Zusammensetzung einen Gehalt an Abbauprodukt(en) des Oxybutynins, insbesondere einen Gehalt an Phenylcyclohexylglykolsäure, von höchstens 10 Gew.-%, insbesondere höchstens 7,5 Gew.-%, vorzugsweise höchstens 5 Gew.-%, bevorzugt höchstens 2 Gew.-%, besonders bevorzugt höchstens 1 Gew.-%, bezogen auf Oxybutyninhydrochlorid, aufweist.

5. Verfahren zur Herstellung einer sterilen oxybutyninhaltigen Zusammensetzung in einer vorzugsweise anwendungsfertigen Kolbenspritze, insbesondere Einweg-Kolbenspritze, und/oder zur Herstellung einer vorzugsweise anwendungsfertigen, sterilen, mit einer oxybutyninhaltigen Zusammensetzung befüllten Kolbenspritze, insbesondere Einweg-Kolbenspritze, insbesondere Verfahren nach einem der vorangehenden Ansprüche,
wobei die Kolbenspritze einen Spritzenkörper, insbesondere Spritzenzylinder, einerseits und einen Spritzenkolben mit einem Kolbenstopfen andererseits aufweist, wobei der Spritzenkörper aus einem Cycloolefin-(Co-)Polymer (COP bzw. COC) gebildet ist und wobei der Kolbenstopfen aus einem Halogenbutylkautschuk, insbesondere aus einem Chlorbutylkautschuk oder aus einem Brombutylkautschuk, vorzugsweise aus einem Brombutylkautschuk, gebildet ist;
wobei das Verfahren die nachfolgenden Schritte umfasst:
a) Befüllen der Kolbenspritze mit der oxybutyninhaltigen Zusammensetzung und/oder Bereitstellen der oxybutyninhaltigen Zusammensetzung in der Kolbenspritze,
wobei das Oxybutynin in Form des Hydrochlorids eingesetzt wird, die oxybutyninhaltige Zusammensetzung als wässrige Zusammensetzung ausgebildet wird und die oxybutyninhaltige Zusammensetzung in Kombination und jeweils in wirksamen, insbesondere pharmazeutisch wirksamen Mengen,
- Oxybutyninhydrochlorid (Oxybutynin-HCI) in einer Konzentration von (1 ± 0,4) mg/ml, insbesondere in einer Konzentration von (1 ± 0,3) mg/ml, vorzugsweise in einer Konzentration von (1 ± 0,2) mg/ml, bevorzugt in einer Konzentration von (1 ± 0,1) mg/ml, besonders bevorzugt in einer Konzentration von etwa 1 mg/ml, und
- Natriumchlorid, berechnet als Natrium, in einer Konzentration von (3,5 ± 0,4) mg/ml, insbesondere in einer Konzentration von (3,5 ± 0,3) mg/ml, vorzugsweise in einer Konzentration von (3,5 ± 0,2) mg/ml, bevorzugt in einer Konzentration von (3,5 ± 0,1) mg/ml, besonders bevorzugt in einer Konzentration von etwa 3,5 mg/ml
enthält,
wobei die oxybutyninhaltige Zusammensetzung auf einen pH-Wert im Bereich von 3 bis 5, insbesondere im Bereich von 3,8 bis 4,5, eingestellt wird und/oder wobei die oxybutyninhaltige Zusammensetzung einen pH-Wert im Bereich von 3 bis 5, insbesondere im Bereich von 3,8 bis 4,5, aufweist;
und nachfolgend
b) Dampfsterilisation, insbesondere Wasserdampfsterilisation, der oxybutyninhaltigen Zusammensetzung in der Kolbenspritze und/oder der mit der oxybutyninhaltigen Zusammensetzung befüllten Kolbenspritze.

6. Verfahren zur Herstellung einer sterilen oxybutyninhaltigen Zusammensetzung in einer vorzugsweise anwendungsfertigen Kolbenspritze, insbesondere Einweg-Kolbenspritze, und/oder zur Herstellung einer vorzugsweise anwendungsfertigen, sterilen, mit einer oxybutyninhaltigen Zusammensetzung befüllten Kolbenspritze, insbesondere Einweg-Kolbenspritze, insbesondere Verfahren nach einem der vorangehenden Ansprüche,
wobei die Kolbenspritze einen Spritzenkörper, insbesondere Spritzenzylinder, einerseits und einen Spritzenkolben mit einem Kolbenstopfen andererseits aufweist, wobei der Spritzenkörper aus einem Cycloolefin-(Co-)Polymer (COP bzw. COC) gebildet ist und wobei der Kolbenstopfen aus einem Halogenbutylkautschuk, insbesondere aus einem Chlorbutylkautschuk oder aus einem Brombutylkautschuk, vorzugsweise aus einem Brombutylkautschuk, gebildet ist;
wobei das Verfahren die nachfolgenden Schritte umfasst:
a) Befüllen der Kolbenspritze mit der oxybutyninhaltigen Zusammensetzung und/oder Bereitstellen der oxybutyninhaltigen Zusammensetzung in der Kolbenspritze,
wobei das Oxybutynin in Form des Hydrochlorids eingesetzt wird, die oxybutyninhaltige Zusammensetzung als wässrige Zusammensetzung ausgebildet wird und die oxybutyninhaltige Zusammensetzung in Kombination und jeweils in wirksamen, insbesondere pharmazeutisch wirksamen Mengen,
- Oxybutyninhydrochlorid (Oxybutynin-HCI) in einer Konzentration von etwa 1 mg/ml, und
- Natriumchlorid, berechnet als Natrium, in einer Konzentration von etwa 3,5 mg/ml
enthält,
wobei die oxybutyninhaltige Zusammensetzung auf einen pH-Wert im Bereich von 3,8 bis 4,5 eingestellt wird und/oder wobei die oxybutyninhaltige Zusammensetzung einen pH-Wert im Bereich von 3,8 bis 4,5 aufweist;
und nachfolgend
b) Dampfsterilisation, insbesondere Wasserdampfsterilisation, der oxybutyninhaltigen Zusammensetzung in der Kolbenspritze und/oder der mit der oxybutyninhaltigen Zusammensetzung befüllten Kolbenspritze,
wobei die Dampfsterilisation unter *overkill*-Bedingungen und/oder bei etwa 121 °C und/oder für einen Zeitraum von mindestens 15 min, insbesondere von mindestens 20 min, vorzugsweise von etwa 20 min, durchgeführt wird und/oder
wobei die Dampfsterilisation gemäß Pharmacopoea Europaea (Ph. Eur.), Kapitel 5.1.1 (07/2017:50101) durchgeführt wird, insbesondere unter *overkill*-Bedingungen und/oder bei etwa 121 °C und/oder für einen Zeitraum von mindestens 15 min, insbesondere von mindestens 20 min, vorzugsweise von etwa 20 min.

7. Sterile oxybutyninhaltige Zusammensetzung, insbesondere zur Verwendung bei der prophylaktischen und/oder therapeutischen Behandlung von neurogenen Blasenfunktionsstörungen, insbesondere neurogenen Blasenentleerungsstörungen, vorzugsweise von mit einer Detrusorhyperaktivität und/oder einer Detrusor-Sphinkter-Dyssynergie einhergehenden neurogenen Blasenfunktionsstörungen,
wobei die oxybutyninhaltige Zusammensetzung nach einem Verfahren gemäß einem der Ansprüchen 1 bis 6 erhältlich ist; und/oder wobei die oxybutyninhaltige Zusammensetzung nach einem Verfahren gemäß einem der Ansprüchen 1 bis 6 dampfsterilisiert, insbesondere wasserdampfsterilisiert, worden ist.

8. Sterile oxybutyninhaltige Zusammensetzung, insbesondere zur Verwendung bei der prophylaktischen und/oder therapeutischen Behandlung von neurogenen Blasenfunktionsstörungen, insbesondere neurogenen Blasenentleerungsstörungen, vorzugsweise von mit einer Detrusorhyperaktivität und/oder einer Detrusor-Sphinkter-Dyssynergie einhergehenden neurogenen Blasenfunktionsstörungen, insbesondere Zusammensetzung nach Anspruch 7,
wobei die oxybutyninhaltige Zusammensetzung in einer vorzugsweise anwendungsfertigen Kolbenspritze, insbesondere Einweg-Kolbenspritze, vorliegt,
wobei die Kolbenspritze einen Spritzenkörper, insbesondere Spritzenzylinder, einerseits und einen Spritzenkolben mit einem Kolbenstopfen andererseits aufweist, wobei der Spritzenkörper aus einem Cycloolefin-(Co-)Polymer (COP bzw. COC) gebildet ist und wobei der Kolbenstopfen aus einem Halogenbutylkautschuk gebildet ist;
wobei die oxybutyninhaltige Zusammensetzung das Oxybutynin in Form des Hydrochlorids enthält, die oxybutyninhaltige Zusammensetzung als wässrige Zusammensetzung ausgebildet ist und die oxybutyninhaltige Zusammensetzung in Kombination und jeweils in wirksamen, insbesondere pharmazeutisch wirksamen Mengen,
- Oxybutyninhydrochlorid (Oxybutynin-HCI) in einer Konzentration von (1 ± 0,5) mg/ml und
- Natriumchlorid, berechnet als Natrium, in einer Konzentration von (3,5 ± 0,5) mg/ml
enthält,
wobei die oxybutyninhaltige Zusammensetzung auf einen pH-Wert im Bereich von 3 bis 5, insbesondere im Bereich von 3,8 bis 4,5, eingestellt ist und/oder wobei die oxybutyninhaltige Zusammensetzung einen pH-Wert im Bereich von 3 bis 5, insbesondere im Bereich von 3,8 bis 4,5, aufweist; und
wobei die in der Kolbenspritze enthaltene oxybutyninhaltige Zusammensetzung einschließlich der Kolbenspritze dampfsterilisiert, insbesondere wasserdampfsterilisiert, ist.

9. Zusammensetzung nach Anspruch 7 oder 8,
wobei die oxybutyninhaltige Zusammensetzung das Oxybutyninhydrochlorid (Oxybutynin-HCI) in einer Konzentration von (1 ± 0,4) mg/ml, insbesondere in einer Konzentration von (1 ± 0,3) mg/ml, vorzugsweise in einer Konzentration von (1 ± 0,2) mg/ml, bevorzugt in einer Konzentration von (1 ± 0,1) mg/ml, besonders bevorzugt in einer Konzentration von etwa 1 mg/ml, enthält; und/oder
wobei die oxybutyninhaltige Zusammensetzung das Natriumchlorid, berechnet als Natrium, in einer Konzentration von (3,5 ± 0,4) mg/ml, insbesondere in einer Konzentration von (3,5 ± 0,3) mg/ml, vorzugsweise in einer Konzentration von (3,5 ± 0,2) mg/ml, bevorzugt in einer Konzentration von (3,5 ± 0,1) mg/ml, besonders bevorzugt in einer Konzentration von etwa 3,5 mg/ml, enthält.

10. Zusammensetzung nach einem der Ansprüche 7 bis 9,
wobei die oxybutyninhaltige Zusammensetzung unter mindestens einer der in den Ansprüchen 1 bis 6 definierten Bedingungen dampfsterilisiert ist; und/oder
wobei die oxybutyninhaltige Zusammensetzung unter *overkill*-Bedingungen und/oder bei etwa 121 °C und/oder für einen Zeitraum von mindestens 15 min, insbesondere von mindestens 20 min, vorzugsweise von etwa 20 min, dampfsterilisiert ist; und/oder
wobei die oxybutyninhaltige Zusammensetzung gemäß Pharmacopoea Europaea (Ph. Eur.), Kapitel 5.1.1 (07/2017:50101) dampfsterilisiert ist, insbesondere unter *overkill*-Bedingungen und/oder bei etwa 121 °C und/oder für einen Zeitraum von mindestens 15 min, insbesondere von mindestens 20 min, vorzugsweise von etwa 20 min; und/oder
wobei die nach Dampfsterilisation erhaltene sterile oxybutyninhaltige Zusammensetzung und/oder die mit der oxybutyninhaltigen Zusammensetzung befüllte nach Dampfsterilisation erhaltene sterile Kolbenspritze einen SAL-Wert (*Sterility* Assurance) von mindestens 10⁻⁵, insbesondere mindestens 10⁻⁶, vorzugsweise mindestens 10⁻⁷, aufweist; und/oder
wobei die nach Dampfsterilisation erhaltene sterile oxybutyninhaltige Zusammensetzung einen Gehalt an Abbauprodukt(en) des Oxybutynins, insbesondere einen Gehalt an Phenylcyclohexylglykolsäure, von höchstens 10 Gew.-%, insbesondere höchstens 7,5 Gew.-%, vorzugsweise höchstens 5 Gew.-%, bevorzugt höchstens 2 Gew.-%, besonders bevorzugt höchstens 1 Gew.-%, bezogen auf Oxybutyninhydrochlorid, aufweist.

11. Sterile oxybutyninhaltige Zusammensetzung, insbesondere zur Verwendung bei der prophylaktischen und/oder therapeutischen Behandlung von neurogenen Blasenfunktionsstörungen, insbesondere neurogenen Blasenentleerungsstörungen, vorzugsweise von mit einer Detrusorhyperaktivität und/oder einer Detrusor-Sphinkter-Dyssynergie einhergehenden neurogenen Blasenfunktionsstörungen, insbesondere Zusammensetzung nach einem der Ansprüche 7 bis 10,
wobei die oxybutyninhaltige Zusammensetzung in einer vorzugsweise anwendungsfertigen Kolbenspritze, insbesondere Einweg-Kolbenspritze, vorliegt,
wobei die Kolbenspritze einen Spritzenkörper, insbesondere Spritzenzylinder, einerseits und einen Spritzenkolben mit einem Kolbenstopfen andererseits aufweist, wobei der Spritzenkörper aus einem Cycloolefin-(Co-)Polymer (COP bzw. COC) gebildet ist und wobei der Kolbenstopfen aus einem Halogenbutylkautschuk, insbesondere aus einem Chlorbutylkautschuk oder Brombutylkautschuk, vorzugsweise aus einem Brombutylkautschuk, gebildet ist;
wobei die oxybutyninhaltige Zusammensetzung das Oxybutynin in Form des Hydrochlorids enthält, die oxybutyninhaltige Zusammensetzung als wässrige Zusammensetzung ausgebildet ist und die oxybutyninhaltige Zusammensetzung in Kombination und jeweils in wirksamen, insbesondere pharmazeutisch wirksamen Mengen,
- Oxybutyninhydrochlorid (Oxybutynin-HCI) in einer Konzentration von (1 ± 0,4) mg/ml, insbesondere in einer Konzentration von (1 ± 0,3) mg/ml, vorzugsweise in einer Konzentration von (1 ± 0,2) mg/ml, bevorzugt in einer Konzentration von (1 ± 0,1) mg/ml, besonders bevorzugt in einer Konzentration von etwa 1 mg/ml, und
- Natriumchlorid, berechnet als Natrium, in einer Konzentration von (3,5 ± 0,4) mg/ml, insbesondere in einer Konzentration von (3,5 ± 0,3) mg/ml, vorzugsweise in einer Konzentration von (3,5 ± 0,2) mg/ml, bevorzugt in einer Konzentration von (3,5 ± 0,1) mg/ml, besonders bevorzugt in einer Konzentration von etwa 3,5 mg/ml,
enthält,
wobei die oxybutyninhaltige Zusammensetzung auf einen pH-Wert im Bereich von 3 bis 5, insbesondere im Bereich von 3,8 bis 4,5, eingestellt ist und/oder wobei die oxybutyninhaltige Zusammensetzung einen pH-Wert im Bereich von 3 bis 5, insbesondere im Bereich von 3,8 bis 4,5, aufweist; und
wobei die in der Kolbenspritze enthaltene oxybutyninhaltige Zusammensetzung einschließlich der Kolbenspritze dampfsterilisiert, insbesondere wasserdampfsterilisiert, ist, insbesondere unter den zuvor definierten Bedingungen.

12. Sterile oxybutyninhaltige Zusammensetzung, insbesondere zur Verwendung bei der prophylaktischen und/oder therapeutischen Behandlung von neurogenen Blasenfunktionsstörungen, insbesondere neurogenen Blasenentleerungsstörungen, vorzugsweise von mit einer Detrusorhyperaktivität und/oder einer Detrusor-Sphinkter-Dyssynergie einhergehenden neurogenen Blasenfunktionsstörungen, insbesondere Zusammensetzung nach einem der Ansprüche 7 bis 11,
wobei die oxybutyninhaltige Zusammensetzung in einer vorzugsweise anwendungsfertigen Kolbenspritze, insbesondere Einweg-Kolbenspritze, vorliegt,
wobei die Kolbenspritze einen Spritzenkörper, insbesondere Spritzenzylinder, einerseits und einen Spritzenkolben mit einem Kolbenstopfen andererseits aufweist, wobei der Spritzenkörper aus einem Cycloolefin-(Co-)Polymer (COP bzw. COC) gebildet ist und wobei der Kolbenstopfen aus einem Halogenbutylkautschuk, insbesondere aus einem Chlorbutylkautschuk oder aus einem Brombutylkautschuk, vorzugsweise aus einem Brombutylkautschuk, gebildet ist;
wobei die oxybutyninhaltige Zusammensetzung das Oxybutynin in Form des Hydrochlorids enthält, die oxybutyninhaltige Zusammensetzung als wässrige Zusammensetzung ausgebildet ist und die oxybutyninhaltige Zusammensetzung in Kombination und jeweils in wirksamen, insbesondere pharmazeutisch wirksamen Mengen,
- Oxybutyninhydrochlorid (Oxybutynin-HCI) in einer Konzentration von etwa 1 mg/ml und
- Natriumchlorid, berechnet als Natrium, in einer Konzentration von etwa 3,5 mg/ml
enthält,
wobei die oxybutyninhaltige Zusammensetzung auf einen pH-Wert im Bereich von 3,8 bis 4,5, eingestellt ist und/oder wobei die oxybutyninhaltige Zusammensetzung einen pH-Wert im Bereich von 3,8 bis 4,5 aufweist; und
wobei die in der Kolbenspritze enthaltene oxybutyninhaltige Zusammensetzung einschließlich der Kolbenspritze dampfsterilisiert, insbesondere wasserdampfsterilisiert, ist,
wobei die Dampfsterilisation unter *overkill*-Bedingungen und/oder bei etwa 121 °C und/oder für einen Zeitraum von mindestens 15 min, insbesondere von mindestens 20 min, vorzugsweise von etwa 20 min, durchgeführt wird und/oder
wobei die Dampfsterilisation gemäß Pharmacopoea Europaea (Ph. Eur.), Kapitel 5.1.1 (07/2017:50101) durchgeführt wird, insbesondere unter *overkill-*Bedingungen und/oder bei etwa 121 °C und/oder für einen Zeitraum von mindestens 15 min, insbesondere von mindestens 20 min, vorzugsweise von etwa 20 min.

13. Kolbenspritze, insbesondere Einweg-Kolbenspritze, wobei die Kolbenspritze einen Spritzenkörper, insbesondere Spritzenzylinder, einerseits und einen Spritzenkolben mit einem Kolbenstopfen andererseits aufweist, wobei der Spritzenkörper aus einem Cycloolefin-(Co-)Polymer (COP bzw. COC) gebildet ist und wobei der Kolbenstopfen aus einem Halogenbutylkautschuk gebildet ist,
wobei die Kolbenspritze mit einer sterilen oxybutyninhaltigen Zusammensetzung, insbesondere zur Verwendung bei der prophylaktischen und/oder therapeutischen Behandlung von neurogenen Blasenfunktionsstörungen, insbesondere neurogenen Blasenentleerungsstörungen, vorzugsweise von mit einer Detrusorhyperaktivität und/oder einer Detrusor-Sphinkter-Dyssynergie einhergehenden neurogenen Blasenfunktionsstörungen, insbesondere wie in einem der Ansprüche 7 bis 12 definiert, befüllt ist,
wobei die Kolbenspritze nach einem Verfahren gemäß einem der Ansprüchen 1 bis 6 erhältlich ist und/oder wobei die in der Kolbenspritze enthaltene oxybutyninhaltige Zusammensetzung einschließlich der Kolbenspritze nach einem Verfahren gemäß einem der Ansprüchen 1 bis 6 dampfsterilisiert, insbesondere wasserdampfsterilisiert, worden ist.

14. Kolbenspritze, insbesondere Einweg-Kolbenspritze, wobei die Kolbenspritze einen Spritzenkörper, insbesondere Spritzenzylinder, einerseits und einen Spritzenkolben mit einem Kolbenstopfen andererseits aufweist, wobei der Spritzenkörper aus einem Cycloolefin-(Co-)Polymer (COP bzw. COC) gebildet ist und wobei der Kolbenstopfen aus einem Halogenbutylkautschuk, gebildet ist, insbesondere Kolbenspritze nach Anspruch 13,
wobei die Kolbenspritze mit einer sterilen oxybutyninhaltigen Zusammensetzung, insbesondere zur Verwendung bei der prophylaktischen und/oder therapeutischen Behandlung von neurogenen Blasenfunktionsstörungen, insbesondere neurogenen Blasenentleerungsstörungen, vorzugsweise von mit einer Detrusorhyperaktivität und/oder einer Detrusor-Sphinkter-Dyssynergie einhergehenden neurogenen Blasenfunktionsstörungen, befüllt ist,
wobei die oxybutyninhaltige Zusammensetzung das Oxybutynin in Form des Hydrochlorids enthält, die oxybutyninhaltige Zusammensetzung als wässrige Zusammensetzung ausgebildet ist und die oxybutyninhaltige Zusammensetzung in Kombination und jeweils in wirksamen, insbesondere pharmazeutisch wirksamen Mengen,
- Oxybutyninhydrochlorid (Oxybutynin-HCI) in einer Konzentration von (1 ± 0,5) mg/ml und
- Natriumchlorid, berechnet als Natrium, in einer Konzentration von (3,5 ± 0,5) mg/ml
enthält,
wobei die oxybutyninhaltige Zusammensetzung auf einen pH-Wert im Bereich von 3 bis 5, insbesondere im Bereich von 3,8 bis 4,5, eingestellt ist und/oder wobei die oxybutyninhaltige Zusammensetzung einen pH-Wert im Bereich von 3 bis 5, insbesondere im Bereich von 3,8 bis 4,5, aufweist; und
wobei die in der Kolbenspritze enthaltene oxybutyninhaltige Zusammensetzung einschließlich der Kolbenspritze dampfsterilisiert, insbesondere wasserdampfsterilisiert, ist;
insbesondere wobei die Kolbenspritze ein Volumen, insbesondere ein Aufnahmevolumen für die oxybutyninhaltige Zusammensetzung, im Bereich von 4 ml bis 25 ml, insbesondere im Bereich von 6 ml bis 20 ml, vorzugsweise im Bereich von 8 ml bis 15 ml, bevorzugt im Bereich von 9 ml bis 12 ml, besonders bevorzugt etwa 10 ml, aufweist; und/oder insbesondere wobei die Kolbenspritze die oxybutyninhaltige Zusammensetzung in einer Menge im Bereich von 4 ml bis 25 ml, insbesondere im Bereich von 6 ml bis 20 ml, vorzugsweise im Bereich von 8 ml bis 15 ml, bevorzugt im Bereich von 9 ml bis 12 ml, besonders bevorzugt von etwa 10 ml, aufweist.

15. Kolbenspritze, insbesondere Einweg-Kolbenspritze, wobei die Kolbenspritze einen Spritzenkörper, insbesondere Spritzenzylinder, einerseits und einen Spritzenkolben mit einem Kolbenstopfen andererseits aufweist, wobei der Spritzenkörper aus einem Cycloolefin-(Co-)Polymer (COP bzw. COC) gebildet ist und wobei der Kolbenstopfen aus einem Halogenbutylkautschuk, insbesondere aus einem Chlorbutylkautschuk oder aus einem Brombutylkautschuk, vorzugsweise aus einem Brombutylkautschuk, gebildet ist, insbesondere Kolbenspritze nach Anspruch 13 oder 14,
wobei die Kolbenspritze mit einer sterilen oxybutyninhaltigen Zusammensetzung, insbesondere zur Verwendung bei der prophylaktischen und/oder therapeutischen Behandlung von neurogenen Blasenfunktionsstörungen, insbesondere neurogenen Blasenentleerungsstörungen, vorzugsweise von mit einer Detrusorhyperaktivität und/oder einer Detrusor-Sphinkter-Dyssynergie einhergehenden neurogenen Blasenfunktionsstörungen, befüllt ist,
wobei die oxybutyninhaltige Zusammensetzung das Oxybutynin in Form des Hydrochlorids enthält, die oxybutyninhaltige Zusammensetzung als wässrige Zusammensetzung ausgebildet ist und die oxybutyninhaltige Zusammensetzung in Kombination und jeweils in wirksamen, insbesondere pharmazeutisch wirksamen Mengen,
- Oxybutyninhydrochlorid (Oxybutynin-HCI) in einer Konzentration von etwa 1 mg/ml und
- Natriumchlorid, berechnet als Natrium, in einer Konzentration von etwa 3,5 mg/ml
enthält,
wobei die oxybutyninhaltige Zusammensetzung auf einen pH-Wert im Bereich von 3,8 bis 4,5 eingestellt ist und/oder wobei die oxybutyninhaltige Zusammensetzung einen pH-Wert im Bereich von 3,8 bis 4,5 aufweist; und
wobei die in der Kolbenspritze enthaltene oxybutyninhaltige Zusammensetzung einschließlich der Kolbenspritze dampfsterilisiert, insbesondere wasserdampfsterilisiert, ist,
wobei die Dampfsterilisation unter *overkill*-Bedingungen und/oder bei etwa 121 °C und/oder für einen Zeitraum von mindestens 15 min, insbesondere von mindestens 20 min, vorzugsweise von etwa 20 min, durchgeführt wird und/oder
wobei die Dampfsterilisation gemäß Pharmacopoea Europaea (Ph. Eur.), Kapitel 5.1.1 (07/2017:50101) durchgeführt wird, insbesondere unter *overkill-*Bedingungen und/oder bei etwa 121 °C und/oder für einen Zeitraum von mindestens 15 min, insbesondere von mindestens 20 min, vorzugsweise von etwa 20 min.

16. Verpackungseinheit, enthaltend mindestens eine Verpackung und mindestens eine Kolbenspritze, insbesondere Einweg-Kolbenspritze, wie in einem der Ansprüche 13 bis 15 definiert, wobei die Kolbenspritze in die Verpackung eingebracht ist und/oder in der Verpackung vorliegt.

17. Kit, insbesondere Applikations- und/oder Instillationssystem, umfassend (i) mindestens eine in einer Kolbenspritze, insbesondere Einweg-Kolbenspritze, insbesondere wie in einem der Ansprüche 13 bis 15 definiert, vorliegende oxybutyninhaltige Zusammensetzung, wie in einem der Ansprüche 7 bis 12 definiert; (ii) (a) mindestens eine an die Kolbenspritze anschließbare Applikations- und/oder Instillationsvorrichtung, insbesondere in Form eines Instillationsschlauches oder dergleichen, vorzugsweise in Form eines (Harnblasen-)Katheters, und/oder (b) mindestens eine an die Kolbenspritze anschließbare Anschlussvorrichtung, insbesondere Adapter, zum Anschließen an eine Applikations- und/oder Instillationsvorrichtung, insbesondere in Form eines Instillationsschlauches oder dergleichen, vorzugsweise in Form eines (Harnblasen-)Katheters; und gegebenenfalls (iii) mindestens eine Applikations- und/oder Instillationsanleitung.

18. Verwendung einer Kolbenspritze, insbesondere Einweg-Kolbenspritze, in einem Verfahren zur Herstellung einer sterilen oxybutyninhaltigen Zusammensetzung, insbesondere wie in einem der Ansprüche 1 bis 6 definiert,
wobei die Kolbenspritze einen Spritzenkörper, insbesondere Spritzenzylinder, einerseits und einen Spritzenkolben mit einem Kolbenstopfen andererseits aufweist, wobei der Spritzenkörper aus einem Cycloolefin-(Co-)Polymer (COP bzw. COC) gebildet ist und wobei der Kolbenstopfen aus einem Halogenbutylkautschuk gebildet ist,
wobei die Kolbenspritze mit der sterilen oxybutyninhaltigen Zusammensetzung, insbesondere zur Verwendung bei der prophylaktischen und/oder therapeutischen Behandlung von neurogenen Blasenfunktionsstörungen, insbesondere neurogenen Blasenentleerungsstörungen, vorzugsweise von mit einer Detrusorhyperaktivität und/oder einer Detrusor-Sphinkter-Dyssynergie einhergehenden neurogenen Blasenfunktionsstörungen, befüllt ist,
wobei die oxybutyninhaltige Zusammensetzung das Oxybutynin in Form des Hydrochlorids enthält, die oxybutyninhaltige Zusammensetzung als wässrige Zusammensetzung ausgebildet ist und die oxybutyninhaltige Zusammensetzung in Kombination und jeweils in wirksamen, insbesondere pharmazeutisch wirksamen Mengen,
- Oxybutyninhydrochlorid (Oxybutynin-HCI) in einer Konzentration von (1 ± 0,5) mg/ml und
- Natriumchlorid, berechnet als Natrium, in einer Konzentration von (3,5 ± 0,5) mg/ml
enthält,
wobei die oxybutyninhaltige Zusammensetzung auf einen pH-Wert im Bereich von 3 bis 5, insbesondere im Bereich von 3,8 bis 4,5, eingestellt ist und/oder wobei die oxybutyninhaltige Zusammensetzung einen pH-Wert im Bereich von 3 bis 5, insbesondere im Bereich von 3,8 bis 4,5, aufweist; und
wobei die in der Kolbenspritze enthaltene oxybutyninhaltige Zusammensetzung einschließlich der Kolbenspritze dampfsterilisiert, insbesondere wasserdampfsterilisiert, wird.

19. Verwendung einer Dampfsterilisation, insbesondere Wasserdampfsterilisation, in einem Verfahren zur Herstellung einer sterilen oxybutyninhaltigen Zusammensetzung in einer vorzugsweise anwendungsfertigen Kolbenspritze, insbesondere Einweg-Kolbenspritze, und/oder zur Herstellung einer vorzugsweise anwendungsfertigen, sterilen, mit einer oxybutyninhaltigen Zusammensetzung befüllten Kolbenspritze, insbesondere Einweg-Kolbenspritze, insbesondere wie in einem der Ansprüche 1 bis 6 definiert,
wobei die Kolbenspritze einen Spritzenkörper, insbesondere Spritzenzylinder, einerseits und einen Spritzenkolben mit einem Kolbenstopfen andererseits aufweist, wobei der Spritzenkörper aus einem Cycloolefin-(Co-)Polymer (COP bzw. COC) gebildet ist und wobei der Kolbenstopfen aus einem Halogenbutylkautschuk gebildet ist;
wobei in der oxybutyninhaltigen Zusammensetzung das Oxybutynin in Form des Hydrochlorids eingesetzt wird, die oxybutyninhaltige Zusammensetzung als wässrige Zusammensetzung ausgebildet wird und die oxybutyninhaltige Zusammensetzung in Kombination und jeweils in wirksamen, insbesondere pharmazeutisch wirksamen Mengen,
- Oxybutyninhydrochlorid (Oxybutynin-HCI) in einer Konzentration von (1 ± 0,5) mg/ml und
- Natriumchlorid, berechnet als Natrium, in einer Konzentration von (3,5 ± 0,5) mg/ml
enthält,
wobei die oxybutyninhaltige Zusammensetzung auf einen pH-Wert im Bereich von 3 bis 5, insbesondere im Bereich von 3,8 bis 4,5, eingestellt wird und/oder wobei die oxybutyninhaltige Zusammensetzung einen pH-Wert im Bereich von 3 bis 5, insbesondere im Bereich von 3,8 bis 4,5, aufweist; und
wobei die mit der oxybutyninhaltigen Zusammensetzung befüllte Kolbenspritze und/oder die in der Kolbenspritze bereitgestellte oxybutyninhaltige Zusammensetzung dampfsterilisiert, insbesondere wasserdampfsterilisiert, wird.
